# EUROPÄISCHE PATENTSCHRIFT

(11) **EP 4 469 020 B1**
(45) Veröffentlichungstag und Bekanntmachung des Hinweises auf die Patenterteilung: **04.02.2026**
(21) Anmeldenummer: 22834484.2
(22) Anmeldetag: 02.12.2022
(51) Int. Cl.: A61K 8/39, A61K 8/86, A61K 8/89, A61K 8/898, A61Q 5/00, A61Q 5/06

(54) **VERFAHREN ZUM BEHANDELN VON KERATINISCHEM MATERIAL UMFASSEND DAS VERMISCHEN VON MINDESTENS ZWEI MITTELN (A) UND (B)**
PROCESS FOR TREATING KERATINOUS MATERIAL, COMPRISING THE MIXING OF AT LEAST TWO AGENTS (A) AND (B)
PROCÉDÉ DE TRAITEMENT DE MATIÈRES KÉRATINIQUES, COMPRENANT LE MÉLANGE D'AU MOINS DEUX AGENTS (A) ET (B)

(30) Priorität: 26.01.2022 DE 102022200856
(43) Veröffentlichungstag der Anmeldung: 04.12.2024
(73) Patentinhaber: Henkel AG & Co. KGaA, 40589 Düsseldorf (DE)
(72) Erfinder: KRUCK, Constanze, 41515 Grevenbroich (DE); MUELLER, Angela, 41515 Grevenbroich (DE); WESER, Gabriele, 45219 Essen (DE)
(86) Internationale Anmeldenummer: PCT/EP2022/084153
(87) Internationale Veröffentlichungsnummer: WO 2023/143780

(56) Entgegenhaltungen:
- WO-A1-2005/063180
- WO-A1-2021/058240
- WO-A1-2021/058241
- WO-A2-2013/085577
- US-B2- 10 682 305

## Beschreibung

Gegenstand der vorliegenden Anmeldung ist ein Verfahren zum Färben von keratinischem Material, insbesondere menschlichen Haaren, welches die Anwendung von mindestens zwei verschiedenen Mitteln (a) und (b) umfasst. Das Mittel (a) enthält mindestens ein aminofunktionalisiertes Silikonpolymer und mindestens ein bei 20 °C flüssiges kosmetisches Öl, und das Mittel (b) beinhaltet Wasser und/oder mindestens ein Alkylenglycol. Vor der Anwendung wird durch Vermischen der Mittel (a) und (b) eine Anwendungsmischung hergestellt, welche auf das Keratinmaterial appliziert, einwirken gelassen und gegebenenfalls wieder abgewaschen wird.

Ein zweiter Gegenstand dieser Anmeldung ist eine Mehrkomponenten-Verpackungseinheit (Kit-of-parts) zum Färben von keratinischem Material, insbesondere menschlichen Haaren, welche getrennt voneinander konfektioniert in zwei verschiedenen Containern die Mittel (a) und (b) umfasst.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Der besondere Vorteil eines Haar-Maskara-Produktes liegt darin, dass die farbgebenden Verbindungen wie beispielsweise Pigmente nur in Form eines Films an der Oberfläche der Keratinfaser abgelagert werden. Die Beschaffenheit der Keratinfaser selbst wird somit bei der Anwendung des Produktes nicht verändert, so dass die Anwendung eines Haar-Maskara-Produktes mit einer besonders geringen Haarschädigung verbunden ist. Wünscht sich der Anwender seine Ursprungshaarfarbe zurück, so kann die Färbung schnell, vollständig und rückstandlos wieder von der Keratinfaser entfernt werden, ohne die Fasern zu schädigen oder die Ursprungshaarfarbe zu verändern. Die Entwicklung von auf Pigmenten basierenden Keratinfärbemitteln liegt daher voll im Trend.

WO2021058241 A1 offenbart ein Verfahren zum Färben von keratinischem Material, insbesondere menschlichen Haaren, umfassend die folgenden Schritte: (1) Bereitstellung eines Mittels (a), wobei das Mittel (a) enthält: (a1) mindestens ein aminofunktionalisiertes Silikonpolymer, (2) Bereitstellung eines Mittels (b), wobei das Mittel (b) enthält: (b1) Wasser und (b2) mindestens einen Fettbestandteil und (b3) mindestens eine farbgebende Verbindung, (3) Herstellung einer Anwendungsmischung durch Vermischen der Mittel (a) und (b), (4) Applizieren der in Schritt (3) hergestellten Anwendungsmischung auf dem keratinischen Material, (5) Einwirken der in Schritt (4) applizierten Anwendungsmischung auf dem keratinischen Material und (6) Ausspülen der Anwendungsmischung mit Wasser.

In aktuellen Arbeiten wurde das Problem der geringen Haltbarkeit dieses Färbesystems adressiert.

In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte. Darüber hinaus konnte durch die Wahl besonders gut geeigneter Pigmente und Pigmentkonzentrationen auf dunklem Haar ein helleres Farbergebnis erzielt werden, so dass mit diesem Färbesystem sogar eine Aufhellung möglich wurde, die bis dato ausschließlich mit oxidativen Haarbehandlungsmitteln (Bleich- bzw. Blondiermitteln) möglich war. Neben diesen vielen Vorteilen besitzt das auf Pigmenten basierende Färbesystem jedoch immer noch einige Nachteile. Insbesondere die Konfektionierung des Aminosilikons hat sich in diesem Zusammenhang als anpruchsvoll erwiesen. Bei Einarbeitung des Aminosilikons zusammen mit Pigmenten in eine wasserhaltige oder lösungsmittelbasierte Formulierung wurde in vielen Fällen eine Lagerinstabilität beobachtet, die optisch in einem Verharzen sichtbar wurde oder auch als Ausbildung vieler kleiner Verklumpung zu beobachten war. Diese entsprechend instabilen Formulierung besaßen nach einem gewissen Lagerzeitraum eine stark verminderte Färbeleistung. Aus diesem Grund wurde dazu übergegangen, Färbemittel mit Pigment und Aminosilikon in getrennt konfektionierten Gebinden im Haarbehandlungsprozess einzusetzen. Das Aminosilikon wurde separat als Konzentrat zur Verfügung gestellt, und für die Haarbehandlung wurde dieses mit einer Trägerformulierung vermischt. Aufgrund der verhältnismäßig kleinen Einsatzmenge war zum einen die vollständige Überführung des Aminosilikons in die Träger- bzw. Färbeformulierung mit Schwierigkeiten verbunden. Darüberhinaus erwies sich auch das Vermischen aus Aminosilikon und Träger- bzw. Färbeformulierung als schwierig. Beispielsweise war ein Verschütteln beider Komponenten in einer Applikationsflasche nicht möglich, da die kleine Menge des Aminosilikons nicht homogen verteilt werden konnte und sich das Aminosilikon zudem während des Verschüttelns in der Applikatorspitze der Flasche absetzte. Bei inhomogener Vermischung des Aminosilikons mit der Färbeformulierung war das im Färbeprozess resultierente Farbresultat entsprechend inhomogen.

Es war die Aufgabe der vorliegenden Erfindung, ein auf Aminosilikonen und Pigmenten basierendes Färbesystem bereitzustellen, das eine gute Lagerstabilität besitzt und intensive Farbresultate mit guten Echtheitseigenschaften bringt. Bei der getrennten Konfektionierung von Aminosilikon und Träger-Formulierung sollte insbesondere eine gute, schnelle, homogene und vollständige Vermischbarkeit der beiden Bestandteile gewährleistet werden, die nach dem Vermischen eine homogene und gleichmäßige Auftragung des Aminosilikons auf dem Haar ermöglicht.

Überraschenderweise hat sich nun herausgestellt, dass die vorgenannte Aufgabe hervorragend gelöst werden kann, wenn keratinische Fasern, insbesondere Haare, mit einem anwendungsbereiten Mittel behandelt wurden, welches vor der Anwendung durch Vermischen von mindestens zwei Mitteln (a) und (b) hergestellt wurde. Hierbei umfasst das Mittel (a) mindestens ein aminofunktionalisiertes Silikonpolymer (a1), und mindestens ein bei 20 °C flüssiges kosmetisches Öl (a2) . Das Öl (a2) dient hierbei der Verdünnung des Aminosilikons (a1), der Vergrößerung des Volumens des Mittels (a) und auch einer Optimierung seiner Fließeigenschaften. Bei dem Vermischen des Mittels (a) mit einer kosmetischen Trägerformulierung (b) konnte auf diesem Wege sowohl eine möglichst vollständige Überführung des Mittels (a) in das Mittel (b) sichergestellt und als auch eine gleichmäßige Vermischung beider Mittel (a) und (b) gewährleistet werden. Auch weitere Mittel wie beispielsweise ein drittes Mittel (c) mit Pigmenten ließ sich sehr gut in die Mischung aus (a) und (b) einarbeiten. Bei Anwendung der Mischung aus (a) und (b) (bzw. der Mischung aus (a) und (b) und (c)) auf Keratinfasern ließ sich das Aminosilikon sehr gleichmäßig auf die Keratinfasern auftragen und bildete dort einen homogenen Film von gleichmäßiger Dicke aus. Die zu dieser Erfindung führenden Arbeiten haben gezeigt, dass diese Methode der Vermischung sowohl in Färbemitteln als auch in anderen Haarbehandlungsmitteln die gleichmäßige Auftragung des Aminosilikons gewährleistet.

Ein erster Gegenstand der vorliegenden Erfindung ist ein Verfahren zur Behandlung von keratinischen Fasern, insbesondere menschlichen Haaren, umfassend die folgenden Schritte:
(1) Bereitstellung eines Mittels (a), wobei das Mittel (a) enthält:
   (a1) mindestens ein aminofunktionalisiertes Silikonpolymer, und
   (a2) mindestens ein bei 20 °C flüssiges kosmetisches Öl, das von (a1) verschieden ist,
(2) Bereitstellung eines Mittels (b), wobei das Mittel (b) enthält:
   (b1) Wasser und/oder mindestens Alkylenglycol der Formel (AG) 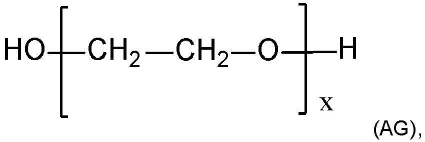 wobei
   - x: für eine ganze Zahl von 1 bis 10000 steht,
(3) gegebenenfalls Bereitstellung eines Mittels (c), wobei das Mittel (c) enthält:
   (c1) mindestens ein Pigment,
(4) Herstellung einer Anwendungsmischung durch Vermischen der Mittel (a) und (b) und gegebenenfalls (c),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) gegebenenfalls Ausspülen der Anwendungsmischung.

### Keratinische Fasern

Unter keratinischen Fasern sind Wolle, Pelze und Federn und insbesondere menschliche Haare zu verstehen. Ganz besonders bevorzugt wird unter keratinischen Fasern das menschliche Haar verstanden.

### Mittel zur Behandlung von keratinischen Fasern

Unter Mitteln zur Behandlung von keratinischen Fasern werden beispielsweise Mittel zur Färbung der Keratinfasern, Mittel zur Umformung oder Formgebung der Keratinfasern oder auch Mittel zur Konditionierung bzw. zur Pflege der Keratinfasern verstanden. Besonders gute Eignung zeigt das erfindungsgemäße Verfahren in Mitteln zur Färbung von keratinischen Fasern, insbesondere menschlichen Haaren.

Der Begriff "Mittel zur Färbung" wird im Rahmen dieser Erfindung für eine durch Einsatz von Pigmenten, hervorgerufene Farbgebung des Keratinmaterials, insbesondere des Haares, verwendet. Bei dieser Färbung lagern sich die Pigmente zusammen mit dem Aminosilikon (a1) in einem besonders homogenen und glatten Film an der Oberfläche der Keratinfasern ab.

### Mittel (a)

In Schritt (1) des erfindungsgemäßen Verfahrens wird das Mittel (a) bereitgestellt. Beispielsweise kann das Mittel (a) in einer Verpackungseinheit oder einem Container vorliegen und auf diese Weise dem Anwender zur Verfügung gestellt werden. Bei dem Container kann es sich beispielsweise um ein Sachet, eine Flasche, eine Dose, einen Tiegel oder auch ein anderes für kosmetische Formulierungen geeignetes Behältnis handeln. Das Mittel (a) ist gekennzeichnet durch seinen Gehalt an mindestens einem aminofunktionalisierten Silikonpolymer (a1) und mindestens einem kosmetischen Öl (a2).

### aminofunktionalisiertes Silikonpolymer (a1) im Mittel (a)

Als erfindungswesentlichen Inhaltsstoff (a1) enthält das Mittel (a) mindestens ein aminofunktionalisiertes Silikonpolymer. Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird auch durch die Polymerisationsmethode mitbestimmt. Im Sinne der vorliegenden Erfindung ist es bevorzugt, wenn das maximale Molekulargewicht des Silikonpolymers nicht mehr als 10⁷ g/mol, bevorzugt nicht mehr als 10⁶ g/mol und besonders bevorzugt nicht mehr als 10⁵ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte mit aminofunktionalisierten Silikonpolymeren (a1) erzielt werden, wenn diese mindestens eine primäre, mindestens eine sekundäre und/oder mindestens eine tertiäre Aminogruppe tragen. Intensive Färbungen mit der besten Waschechtheit wurden jedoch erhalten, wenn ein aminofunktionalisiertes Silikonpolymer (a1) im Mittel (a) eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) mit mindestens eine sekundären Aminogruppe enthält.

Die sekundäre(n) Aminogruppe(n) kann bzw. können sich an verschiedenen Positionen des aminofunktionalisierten Silikonpolymers befinden. Ganz besonders gute Effekte wurden gefunden, wenn ein aminofunktionalisiertes Silikonpolymer (a1) eingesetzt wurde, dass mindestens eine, bevorzugt mehrere Struktureinheiten der Formel (Si-Amino) besitzt.

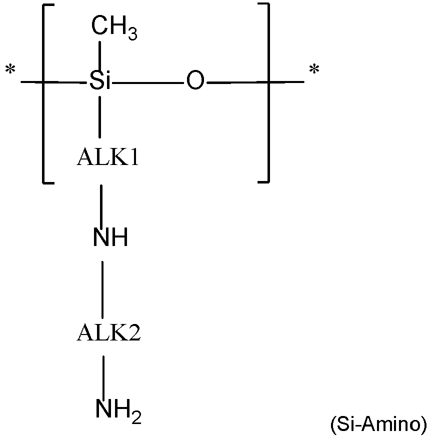

In den Struktureinheiten der Formel (Si-Amino) stehen die Kürzel ALK1 und ALK2 unabhängig voneinander für eine lineare oder verzweigte, zweiwertige C₁-C₂₀-Alkylengruppe.

In einer weiteren ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das mindestens eine Struktureinheit der Formel (Si-Amino) umfasst, 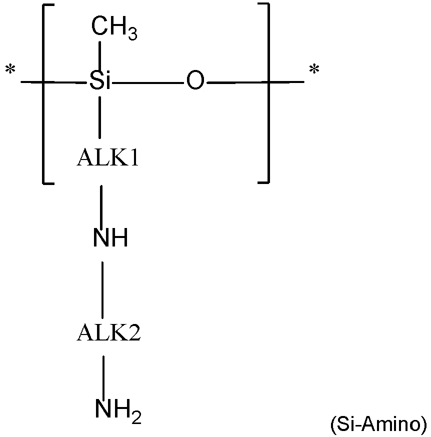 wobei
- ALK1 und ALK2: unabhängig voneinander für eine lineare oder verzweigte, zweiwertige C₁-C₂₀-Alkylengruppe stehen.

Die mit einem Stern (*) gekennzeichneten Positionen geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine C₁-C₆-Alkylgruppe gebunden sein.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In einer weiteren besonders bevorzugten Ausführungsform stellen die Struktureinheiten der Formel (Si-Amino) Wiederholungseinheiten im aminofunktionalisierten Silikonpolymer (a1) dar, so dass das Silikonpolymer mehrere Struktureinheiten der Formel (Si-Amino) umfasst.

Im Folgenden werden besonders gut geeignete aminofunktionalisierte Silikonpolymere (a1) mit mindestens einer sekundären Aminogruppe aufgelistet.

Färbungen mit den allerbesten Waschechtheiten konnten erhalten werden, wenn im erfindungsgemäßen Verfahren mindestens ein Mittel (a) auf dem keratinischen Material appliziert wurde, das mindestens ein aminofunktionalisiertes Silikonpolymer (a1) enthält, das Struktureinheiten der Formel (Si-I) und der Formel (Si-II) umfasst

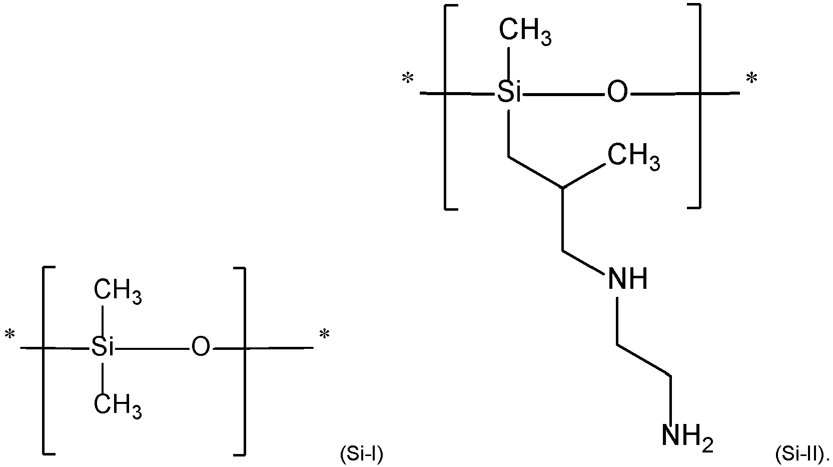

In einer weiteren explizit ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das Struktureinheiten der Formel (Si-I) und der Formel (Si-II) umfasst

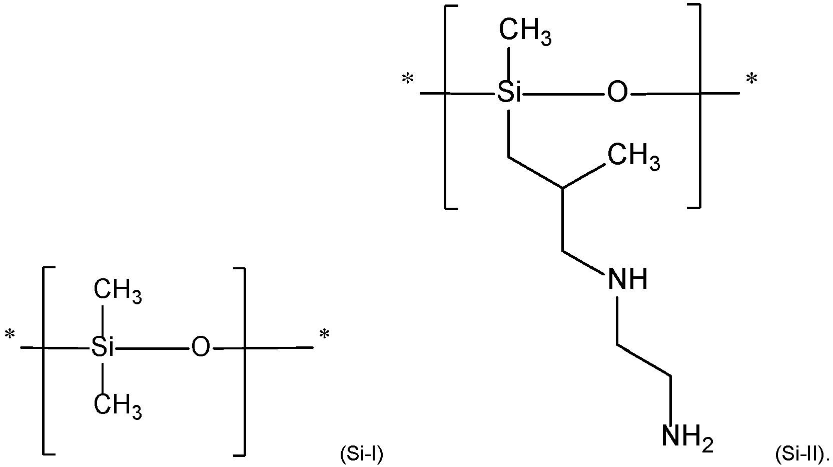

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. Ein weiteres besonders bevorzugtes Handelsprodukt ist Dowsil AP-8568 Amino Fluid, das ebenfalls von der Firma Dow Chemical Company kommerziell vertrieben wird.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel der Formel (Si-III) enthält, 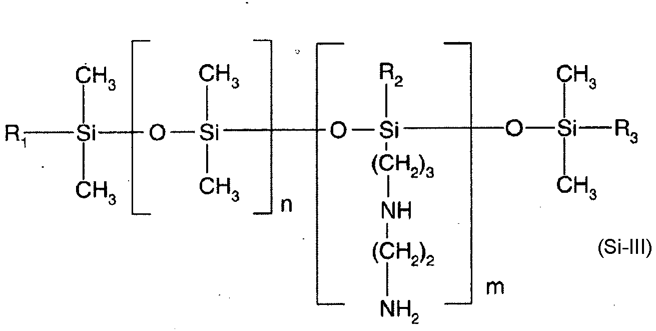 wobei
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die Anwendung eines Mittels (a) auf dem keratinischem Material, wobei das Mittel (a) mindestens aminofunktionelles Silikonpolymer (a1) der Formel der Formel (Si-IV) enthält, 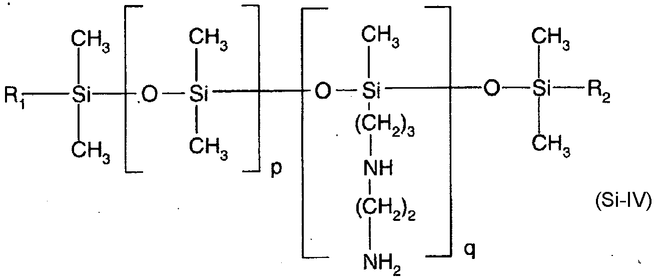 in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-Si(CH₃)₂-Gruppe an eine -[O-Si(CH₃)₂]-Gruppierung gebunden.

Als besonders wirkungsvoll im Hinblick auf die gewünschten Effekte haben sich auch erfindungsgemäßen Verfahren erwiesen, in welchen ein Mittel (a) auf den Keratinfasern appliziert wird, welches mindestens ein aminofunktionelles Silikonpolymer (a1) der Formel der Formel (Si-V) enthält 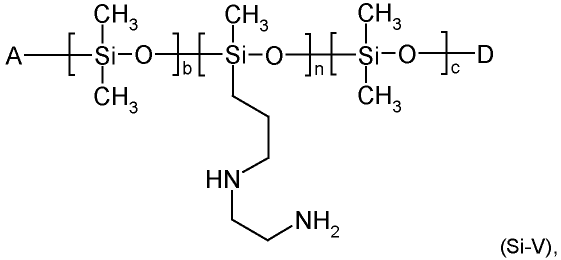 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen,
mit den Maßgaben
- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel (a) kann weiterhin auch ein oder mehrere verschiedene aminofunktionalisierte Silikonpolymere enthalten, die durch die Formel (Si-VI)

M(RₐQ_{b}SiO_{(4-a-b)/2)x}(R_{c}SiO_{(4-c)/2)y}M (Si-VI)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂ )_{z}NH₂, worin z 1 oder mehr ist. Eine andere mögliche Formel für Z ist -NH(CH₂)_{z}(CH ₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO(_{4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die Anwendung eines Mittels (a) auf dem keratinischem Material, wobei das Mittel (a) ein aminofunktionelles Silikonpolymer der Formel (Si-VII)

R'ₐG₃₋ₐ-Si(OSiG ₂)ₙ-(OSiG _{b}R'_{2- b})ₘ-O-SiG₃₋ₐ-R'ₐ (Si-VII),

enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel (Si-Vlla) enthält, 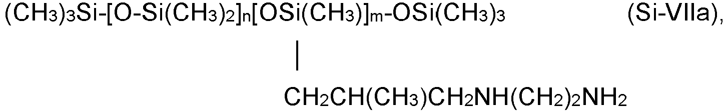 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer der Formel (Si-Vllb) enthält 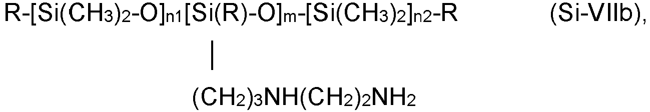 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel (a) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Mittel (a) zum Einsatz im erfindungsgemäßen Verfahren geeignet, welche ein spezielles 4-Morpholinomethyl-substituiertes Silikonpolymer (a1) enthielten. Dieses aminofunktionalisierte Silikonpolymer umfasst Struktureinheiten der Formeln (SI-VIII) und der Formel (Si-IX)

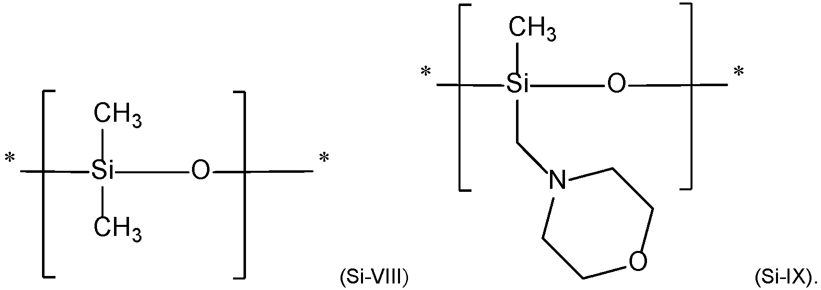

Entsprechende 4-Morpholinomethyl-substituiertes Silikonpolymere werden im folgenden beschrieben.

Ein ganz besonders bevorzugtes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist 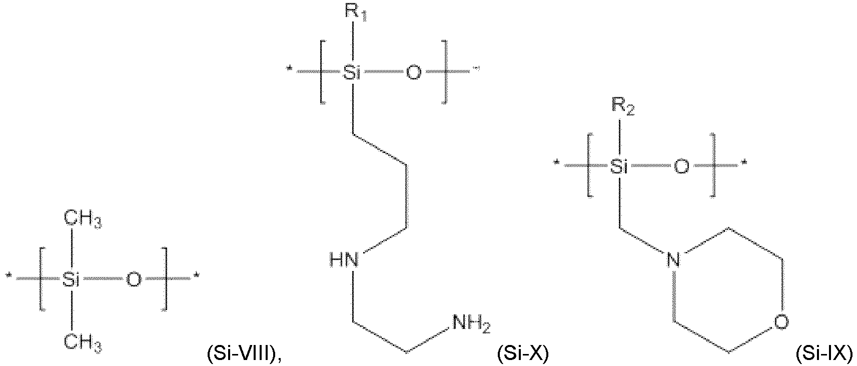 in denen
- R1: für -CH₃, -OH, -OCH₃, -O-CH₂CH₃, -O-CH₂CH₂CH₃, oder -O-CH(CH₃)₂ steht;
- R2: für -CH₃, -OH, oder -OCH₃ steht.
Besonders bevorzugte erfindungsgemäße Mittel (a) enthalten mindestens ein 4-morpholinomethylsubstituierten Silikons der Formel (Si-XI) 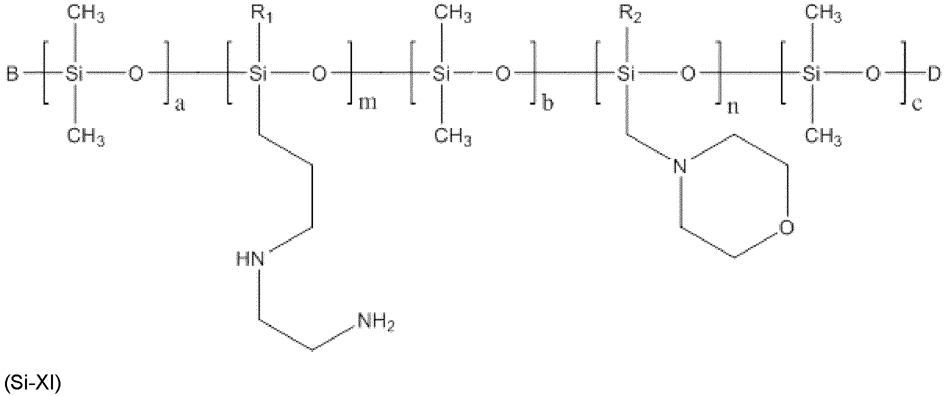 in der
R1 für -CH₃, -OH, -OCH₃, -O-CH₂CH₃, -O-CH₂CH₂CH₃, oder -O-CH(CH₃)₂ steht;
R2 für -CH₃, -OH, oder -OCH₃ steht.
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen
mit den Maßgabe, daß
   - mindestens eine der Bedingungen B = -OH bzw. D = -H erfüllt ist,
   - die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = -O-Si(CH₃)₂OH und | | D = -Si(CH₃)₃ |
| B = -O-Si(CH₃)₂OH und | | D = -Si(CH₃)₂OH |
| B = -O-Si(CH₃)₂OH und | | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Das oder die aminofunkionaliserten Silikonpolymere (a1) werden bevorzugt in bestimmten Mengen im Mittel (a) eingesetzt. Eine besonders gute Vermischbarkeit und auch gute anwendungstechnische Eigenschaften auf den Haaren wurden erhalten, wenn das Mittel (a) - bezogen auf das Gesamtgewicht des Mittels (a) - ein oder mehrere aminofunktionalisierte Silikonpolymere (a1) in einer Gesamtmenge von 1 bis 90 Gew.-%, bevorzugt von 2 bis 70 Gew.-%, weiter bevorzugt von 3 bis 50 Gew.-%, noch weiter bevorzugt von 4 bis 30 Gew.-%, und ganz besonders bevorzugt von 5 bis 20 Gew.-% enthielt.

In einer weiteren explizit ganz besonders bevorzugten Ausführungsform ist ein erfindungsgemäßes Verfahren dadurch gekennzeichnet, dass das Mittel (a) - bezogen auf das Gesamtgewicht des Mittels (a) - ein oder mehrere aminofunktionalisierte Silikonpolymere (a1) in einer Gesamtmenge von 1 bis 90 Gew.-%, bevorzugt von 2 bis 70 Gew.-%, weiter bevorzugt von 3 bis 50 Gew.-%, noch weiter bevorzugt von 4 bis 30 Gew.-%, und ganz besonders bevorzugt von 5 bis 20 Gew.-% enthält.

### Flüssge kosmetische Öle (a2) im Mittel (a)

Als zweiten erfindungswesentlichen Bestandteil (a2) enthält das Mittel (a) mindestens ein bei 20 °C flüssiges kosmetisches Öl. Das bei 20 °C flüssige Öl (a2) dient der Verdünnung des Aminosilikons (a1), vergößert das Volumen des Mittels (a) und verbessert auch die Fließeigenschaften des Mittels (a). Unter einem Öl wird im Sinne der vorliegenden Erfindung eine organische Flüssigkeit verstanden, die bei 20 °C flüssig bzw. fließfähig ist und nicht mit Wasser mischbar ist. Ein Öl ist dann nicht mit Wasser mischbar, wenn es eine Löslichkeit in Wasser bei 20 °C (760 mmHg) von weniger als 1 Gew.-% besitzt.

Die Wasserlöslichkeit des Öls kann beispielsweise auf dem folgenden Weg bestimmt werden. 1,0 g des Öls werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ist in dem Öl-Wasser-Gemisch nach diesem Zeitraum noch eine zweite Phase, d.h. neben der Wasserphase eine separat vorliegende Ölphase (z.B. in Form von Öltröfpchen) erkennbar, dann liegt die Löslichkeit des Öls bei weniger als 1 Gew.-%.

Das Öl (a2) soll die Vermischbarkeit und Homogenisierung des Aminosilikos (a1) verbessern, daher stellt es selbst kein Aminosilikon dar. Aus diesem Grund ist das Öl (a2) von dem aminofunktionalisierten Silikonpolymer (a1) verschieden.

Besonders gut geeingete Öle (a2) können ausgewählt weden aus der Gruppe aus nicht-aminofunktionalisierten Oligoalkylsiloxanen, nicht-aminofunktionalisierten Silikonpolymeren, Paraffinölen, Isoparaffinölen, synthetischen C₃-C₁₂-Kohlenwasserstoffen, Di-C₁₂-C₂₃-Alkylethern, pflanzlichen Ölen und Esterölen

In einer weiteren explizit ganz besonders bevorzugten Ausführungsform ist ein erfindungsgemäßes Verfahren dadurch gekennzeichnet, dass das Mittel (a) mindestens ein bei 20 °C flüssiges kosmetisches Öl (a2) enthält, das ausgewählt ist aus der Gruppe aus nicht-aminofunktionalisierten Oligoalkylsiloxanen, nicht-aminofunktionalisierten Silikonpolymeren, Paraffinölen, Isoparaffinölen, synthetischen C₃-C₁₂-Kohlenwasserstoffen, Di-C₁₂-C₂₃-Alkylethern, pflanzlichen Ölen und Esterölen.

Ganz besonders gut geeignete kosmetische Öle (a2) sind ausgewählt aus der Gruppe aus nicht-aminofunktionalisierten Oligoalkylsiloxanen und nicht-aminofunktionalisierten Silikonpolymeren. Unter Oligoalkylsiloxanen werden im Sinne der Erfindung oligomere Siloxane verstanden, die linear oder cyclisch sein können. Die Oligoalkylsiloxanen sind nicht aminofunktionalisiert, was bedeutet, dass sie keine Aminogruppe in ihrer Struktur tragen.

Bevorzugte lineare Oligoalkylsiloxane sind Verbindungen der allgemeinen Formel (OAS-I) 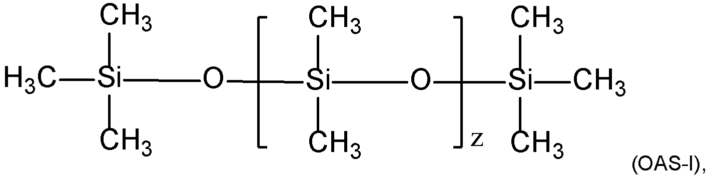 wobei z für eine ganze Zahl von 0 bis 10 steht. Bevorzugt steht z für die Zahlen 0, 1, 2 oder 3.

Ganz besonders bevorzugte lineare Oligoalkylsiloxane sind beispielsweise
- Hexamethyldisiloxan 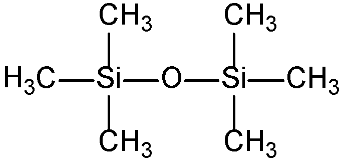
- Octamethyltrisiloxan 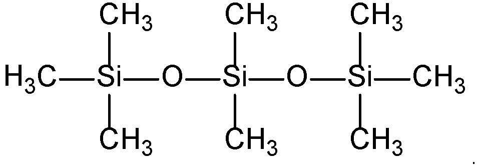
- Decamethyltetrasiloxan

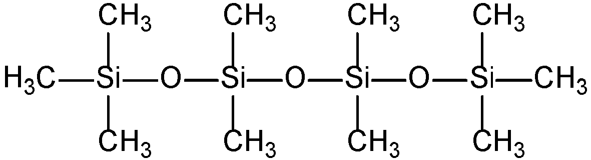

Hexamethyldisiloxan besitzt die CAS-Nummer 107-46-0 und kann beispielsweise bei Sigma-Aldrich kommerziell erworben werden.

Octamethyltrisiloxan besitzt die CAS-Nummer 107-51-7 und ist ebenfalls bei Sigma-Aldrich kommerziell erhältlich.

Decamethyltetrasiloxan trägt die CAS-Nummer 141-62-8 und ist ebenfalls bei Sigma-Aldrich kommerziell erhältlich.

Bevorzugte cyclische Oligoalkylsiloxane sind Verbindungen der allgemeinen Formel (OAS-II) 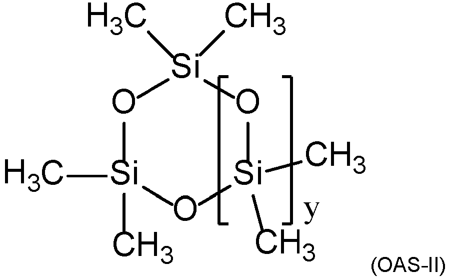 wobei y für eine ganze Zahl von 1 bis 5 steht. Bevorzugt steht z für die Zahlen 1, 2 oder 3.

Ganz besonders bevorzugte cyclische Oligoalkylsiloxane sind beispielsweise Hexamethylcyclotrisiloxan, Octamethylcyclotetrasiloxan und Decamethylcyclopentasiloxan.

In einer weiteren bevorzugten Ausführungsform ist ein erfindungsgemäßes Verfahren dadurch gekennzeichnet, dass das Mittel (a) mindestens ein bei 20 °C flüssiges kosmetisches Öl (a2) enthält, das ausgewählt ist aus der Gruppe der Oligoalkylsiloxane der Formel (OAS-I) und/oder (OAS-II), 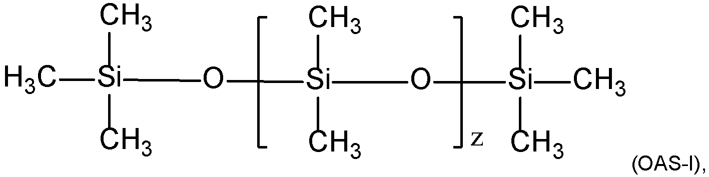 wobei z für eine ganze Zahl von 0 bis 10, bevorzugt für eine ganze Zahl von 0 bis 3, besonders bevorzugt für die Zahl 0 steht, 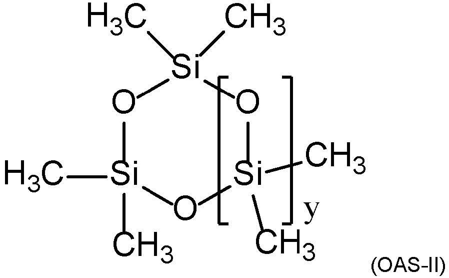 wobei y für eine ganze Zahl von 1 bis 5, bevorzugt für eine ganze Zahl von 1 bis 3, steht.

In einer weiteren bevorzugten Ausführungsform ist ein erfindungsgemäßes Verfahren dadurch gekennzeichnet, dass das Mittel (a) mindestens ein bei 20 °C flüssiges kosmetisches Öl (a2) enthält, das ausgewählt ist aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan.

Die ebenfalls besonders gut zur Lösung der erfindunsggemäßen Aufgabenstellung geeigneten nicht-aminofunktionalisierten Silikonpolymere können alternativ auch als Silikonöle bezeichnet werden. Die Silikonöle sind nicht aminofunktionalisiert, was bedeutet, dass sie keine Aminogruppe in ihrer Struktur tragen.

Silikonöle sind polymere Verbindungen, deren Molekulargewicht bevorzugt bei mindestens 500 g/mol, bevorzugt bei mindestens 1000 g/mol, weiter bevorzugt bei mindestens 2500 g/mol, und besonders bevorzugt von mindestens 5000 g/mol liegt.

Silikonöle umfassen Si-O-Wiederholungseinheiten, wobei die Si-Atome organische Reste wie beispielsweise Alkylgruppen oder substituierte Alkylgruppen tragen können.

In Entsprechung des hohen Molekulargewichts der Silikonöle basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten.

In einer weiteren bevorzugten Ausführungsform ist ein erfindungsgemäßes Verfahren gekennzeichnet, dass das Mittel (a) mindestens ein bei 20 °C flüssiges kosmetisches Öl (a2) enthält, das aus der Gruppe der Polydimethylsiloxane ausgewählt ist.

Es hat sich als ganz besonders bevorzugt erwiesen, im Mittel (a) Silikonöle, insbesondere mit einer Viskosität von 0,5 bis 30000 mm²/s, weiter bevorzugt von 0,5 bis 20000 mm²/s, noch weiter bevorzugt von 0,5 bis 10000 mm²/s, und besonders bevorzugt von 0,5 bis 500 mm²/s, gemessen nach dem ASTM-Standard D-445, einzusetzen.

Der ASTM-Standard D-445 ist das Standard-Verfahren zur Messung der kinematischen Viskosität von transparenten und opaken Flüssigkeiten.

Die Messung der Viskosität erfolgte insbesondere nach ASTM-Standard D-446, Version 06 (D445-06), publiziert Juni 2006. Bei dieser Messmethode wird die Zeit gemessen, welche das definierte Volumen einer Flüssigkeit benötigt, um unter definierten Bedingungen durch die Kappilare eines kalibrierten Viskosimeters zu fließen. Betreffend die Einzelheiten des Verfahrens wird auf ASMT-D445, insbesondere ASTM D445-06 verwiese. Messtemperatur ist 25 °C. Geeignete Geräte (wie Viskosimeter und Thermometer und die entsprechenden Kalibrierungen) sind in der Methode angegeben.

Im Rahmen einer weiteren explizit ganz besonders bevorzugten Ausführungsform ist ein erfindungsgemäßes Verfahren dadurch gekennzeichnet, dass das Mittel (a) mindestens ein Silikonöl mit einer Viskosität von mit einer Viskosität von 0,5 bis 30000 mm²/s, weiter bevorzugt von 0,5 bis 20000 mm²/s, noch weiter bevorzugt von 0,5 bis 10000 mm²/s, und besonders bevorzugt von 0,5 bis 500 mm²/s, gemessen nach dem ASTM-Standard D-445 (25 °C), enthält.

Prinzipiell können im Mittel (c) verschiedene Silikonöle eingesetzt werden, jedoch hat sich der Einsatz von Polydimethylsiloxanen als besonders vorteilhaft im Hinblick auf die Verbesserung des Griffgefühls und die Reduzierung der öligen Haptik der Haare erwiesen.

Aus diesem Grund ist es ganz besonders bevorzugt, wenn das Mittel (a) mindestens ein Silikonöl (a2) aus der Gruppe der Polydimethylsiloxane (Dimethicone) enthält.

Die Silikonöle aus der Gruppe der linearen Polydimethylsiloxane sind Verbindungen der allgemeinen Struktur (PDMS)

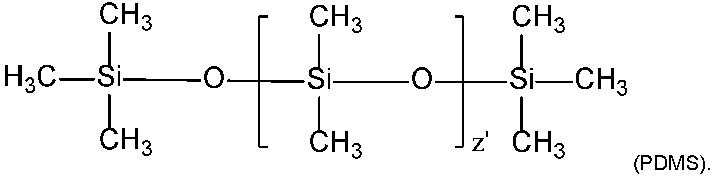

Hierbei wird z' so gewählt, dass die Dimethicone flüssig sind und bevorzugt die vorgenannten ganz besonders gut geeigneten Viskositätsbereiche besitzen.

Bevorzugt kann z' für eine ganze Zahl von 50 bis 100000, weiter bevorzugt von 100 bis 50000, besonders bevorzugt von 500 bis 50000 stehen.

Entsprechende Dimethicone können von verschiedenen Herstellern kommerziell erworben werden. Ganz besonders gut geeignet ist beispielsweise das unter dem Handelsnamen Xiameter PMX 200 Silicone Fluid 50 CS von Dow Chemicals käuflich erwerbliche Dimethicone, dessen Viskosität bei 50 mm²/s liegt (bei 25 °C). Dieses Dimethicone ist am allermeisten bevorzugt.

Ein weiteres besonders gut geeignetes Dimethicone ist das ebenfalls von Dow Corning erhältliche Xiameter PMX 200 Silicone Fluid 100 CS, dessen Viskosität bei 100 mm²/s liegt (Messung bei 25 °C).

Ein weiteres besonders gut geeignetes Dimethicone ist das ebenfalls von Dow Corning erhältliche Xiameter PMX 200 Silicone Fluid 350 CS, dessen Viskosität bei 350 mm²/s liegt (bei 25 °C).

Ein weiteres besonders gut geeignetes Dimethicone ist das von Dow Corning erhältliche Dow Corning 200 fluid 500 cSt, dessen Viskosität bei 500 mm²/s liegt (bei 25 °C).

In einer weiteren besonders bevorzugten Ausführungsform ist ein erfindungsgemäßes Verfahren dadurch gekennzeichnet, dass das Mittel (a) mindestens ein bei 20 °C flüssiges kosmetisches Öl (a2) enthält, das aus der Gruppe der Polydimethylsiloxane ausgewählt ist und das bevorzugt eine Viskosität von 0,5 bis 30000 mm²/s, weiter bevorzugt von 0,5 bis 20000 mm²/s, noch weiter bevorzugt von 0,5 bis 10000 mm²/s, und besonders bevorzugt von 0,5 bis 500 mm²/s, gemessen nach dem ASTM-Standard D-445, (immer gemessen nach dem ASTM-Standard D-445, 25 °C) besitzt.

Weiterhin gut geeignete Öle (a2) sind flüssige Paraffinöle, wie zum Beipsiel Paraffinum Liquidum und Paraffinum Perliquidum, Isoparaffinöle wie beispielsweise Isodecane, synthetische Kohlenwasserstoffe wie zum Beipsiel Undedan und Tridecan, sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Din-dodecylether, n-Hexyl-n-octylether, n-Octyl-n-decylether, n-Decyl-n-undecylether, n-Undecyl- n-dodecylether und n-Hexyl-n-Undecylether sowie Di-tert-butylether, Di-isopentylether, Di-3-ethyldecylether, tert.-Butyl-n-octylether, iso-Pentyl-n-octylether und 2-Methyl-pentyl-n- octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Weitere geeignete kosmetische Öle (a2) können ausgewählt sein aus der Gruppe der pflanzlichen Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.

Weitere geeignete kosmetische Öle (a2) können auch aus der Gruppe der Esteröle ausge-wählt sein. Unter Esterölen sind zu verstehen die Ester von C₆ -C₃₀ -Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure,lsostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isotridecyl Isononanoat, Neopentyl Glycol Diheptanoate, Isopropylmyristat (Rilanit^{®} 1PM), Isononansäure-C16-18-alkylester (Cetiol^{®} SN),2-Ethylhexylpalmitat (Cegesoft^{®} 24),Stearinsäure-2-ethylhexylester (Cetiol^{®} 868), Cetyloleat, Glycerintricaprylat, Kokosfettalkohol- caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat(Cetiol^{®} B), Myristylmyristat (Cetiol^{®} MM), Cetearyllsononanoate (Cetiol^{®} SN) und Ölsäuredecylester (Cetiol^{®} V).

Weitere geeignete kosmetische Öle (a2) können ausgewählt sein aus der Gruppe der Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, symmetrische, unsymmetrische oder cyclische Ester der Kohlensäure mit Fettalkoholen, Glycerincarbonat und Dicaprylylcarbonat (Cetiol^{®} CC).

Das bzw. die kosmetischen Öle (a2) sind im Mittel (a) bevorzugt in bestimmten Mengenbereichen enthalten. Ganz besonders bevorzugt enthält das Mittel (a) - bezogen auf das Gesamtgewicht des Mittels (a) - ein oder mehrere bei 20 °C flüssige kosmetische Öle in einer Gesamtmenge von 1 bis 99 Gew.-%, bevorzugt von 30 bis 98 Gew.-%, weiter bevorzugt von 50 bis 97 Gew.-%, noch weiter bevorzugt von 70 bis 96 Gew.-% und ganz besonders bevorzugt von 80 bis 95 Gew.-%.

Im Rahmen einer weiteren bevorzugten Ausführungsform ist ein erfindungsgemäßes Verfahren dadurch gekennzeichnet, dass das Mittel (a) - bezogen auf das Gesamtgewicht des Mittels (a) - ein oder mehrere bei 20 °C flüssige kosmetische Öle in einer Gesamtmenge von 1 bis 99 Gew.-%, bevorzugt von 30 bis 98 Gew.-%, weiter bevorzugt von 50 bis 97 Gew.-%, noch weiter bevorzugt von 70 bis 96 Gew.-% und ganz besonders bevorzugt von 80 bis 95 Gew.-% enthält.

Im Rahmen einer weiteren bevorzugten Ausführungsform ist ein erfindungsgemäßes Verfahren dadurch gekennzeichnet, dass das Mittel (a) - bezogen auf das Gesamtgewicht des Mittels (a) - ein oder mehrere nicht-aminofunktionalisierten Oligoalkylsiloxane in einer Gesamtmenge von 1 bis 99 Gew.-%, bevorzugt von 30 bis 98 Gew.-%, weiter bevorzugt von 50 bis 97 Gew.-%, noch weiter bevorzugt von 70 bis 96 Gew.-% und ganz besonders bevorzugt von 80 bis 95 Gew.-% enthält.

Im Rahmen einer weiteren bevorzugten Ausführungsform ist ein erfindungsgemäßes Verfahren dadurch gekennzeichnet, dass das Mittel (a) - bezogen auf das Gesamtgewicht des Mittels (a) - ein oder mehrere Silikonöle aus der Gruppe der Dimethicone in einer Gesamtmenge von 1 bis 99 Gew.-%, bevorzugt von 30 bis 98 Gew.-%, weiter bevorzugt von 50 bis 97 Gew.-%, noch weiter bevorzugt von 70 bis 96 Gew.-% und ganz besonders bevorzugt von 80 bis 95 Gew.-% enthält.

### Verhältnisse von (a1) zu (a2) im Mittel (a)

Zur Optimierung der anwendungstechnischen Eigenschaften enthält das Mittel (a) die Bestandteile (a1) und (a2) besonders bevorzugt in bestimmten Gewichtsverhältnissen zueinander. Bei den zu dieser Erfindung führenden Arbeiten wurde beobachtet, dass die Einstellung des Gewichtsverhältnisses aus den im Mittel enthaltenen flüssigen Ölen (a2) zu den im Mittel (a) enthaltenen Amnosilikonen (a1) zu einer bestmöglichen Vermischung, einer guten Lagerstabilität und sehr guten Ergebnissen bei einer anschließenden Färbung führte.

Besonders gute Ergebnisse wurden erhalten, wenn das Mittel (a) ein oder mehrere kosmetische Öle (a2) und ein oder mehrere aminofunktionalisierte Silikonpolymere (a1), d.h. die Bestandteile (a2)/(a1), in einem Gewichtsverhältnis von 100:1 bis 1:100, bevorzugt von 100:1 bis 1:1, weiter bevorzugt von 50:1 bis 2:1, noch weiter bevorzugt von 40:1 bis 4:1 und ganz besonders bevorzugt von 20:1 bis 4:1 enthielt.

In einer weiteren besonders bevorzugten Ausführungsform ist ein erfindungsgemäßes Verfahren dadurch gekennzeichnet, dass das Mittel (a) die kosmetischen Öle (a2) und die aminofunktionalisierten Silikonpolymere (a1), d.h. die Bestandteile (a2)/(a1), in einem Gewichtsverhältnis von 100:1 bis 1:100, bevorzugt von 100:1 bis 1:1, weiter bevorzugt von 50:1 bis 2:1, noch weiter bevorzugt von 40:1 bis 4:1 und ganz besonders bevorzugt von 20:1 bis 4:1 enthält.

Anders ausgedrückt konnte herausgefunden werden, dass sowohl eine ganz besonders gute Vermischung als auch eine homogene Färbung erzielt wurde, wenn das Mittel (a) die kosmetischen Öle (a2) im Vergleich zu den Aminosilikonen (a1) in einem 4-fachen bis 20-fachen Gewichtsüberschuss enthielt.

### Mittel (b)

In Schritt (2) des erfindungsgemäßen Verfahrens wird ein kosmetisches Mittel (b) bereitgestellt. Wie das Mittel (a) kann auch das Mittel (b) dem Anwender bevorzugt in einer Verpackungseinheit oder einem Container vorliegen und auf diese Weise dem Anwender zur Verfügung gestellt werden. Bei dem Container kann es sich beispielsweise um ein Sachet, eine Flasche, eine Dose, einen Tiegel oder auch ein anderes für kosmetische Formulierungen geeignetes Behältnis handeln.

Das Mittel (b) stellt eine kosmetische Trägerformulierung dar, und ist dadurch gekennzeichnet, dass dieser kosmetische Träger entweder Wasser oder ein Alkylenglycol der Formel (AG) ist, 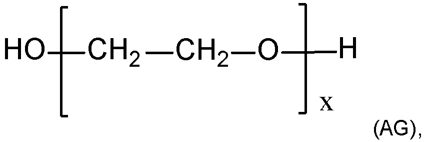 wobei
- x: für eine ganze Zahl von 1 bis 10000 steht.

Auch eine Mischung aus Wasser und Alkylenglcyol ist als kosmetischer Träger geeignet, und in diesem Fall enthält das Mittel (b) sowohl Wasser als auch Alkylenglycol der Formel (AG).

Bei den Alkylenglycolen der Formel (AG) handelt es sich um protische Substanzen mit mindestens zwei Hydroxy-Gruppen, die aufgrund ihrer Wiederholungseinheit -CH2-CH2-O- , sofern x für einen Wert von mindestens 2 steht, auch als Polyethylenglycole bezeichnet werden können. In den Alkylenglycolen (b1) der Formel (AG) steht x für eine ganze Zahl von 1 bis 10000. Im Rahmen der zu dieser Erfindung führenden Arbeiten hat sich herausgestellt, dass diese Polyethylenglycole eine besonders gute Eignung zeigen, um zum einen die Echtheitseigenschaften der Färbemittel zu verbessern und zum anderen auch um die Viskosität der Mittel optimal einzustellen.

Abhängig von ihrer Kettenlänge sind Polyethylenglycole flüssige oder feste, wasserlösliche Polymere. Polyethylenglycole mit einer Molekülmasse zwischen 200 g/mol und 400 g/mol sind bei Raumtemperatur nichtflüchtige Flüssigkeiten. PEG 600 weist einen Schmelzbereich von 17 bis 22 °C und somit eine pastenartige Konsistenz auf. Bei Molekülmassen über 3000 g/mol sind die PEG feste Substanzen und werden als Schuppen oder Pulver in den Handel gebracht.

Vor allem der Einsatz von niedermolekularen Alkylenglycolen (bzw. Polyethylenglycolen) hat sich zur Lösung der erfindungsgemäßen Aufgabenstellung gut geeignet erwiesen. Bei niedermolekularen Alkylenglycolen (bzw. Polyethylenglycolen) im Sinne der vorliegenden Erfindung steht x für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15.

Im Rahmen einer weiteren ganz besonders bevorzugten Ausführungsform ist ein erfindungsgemäßes Verfahren dadurch gekennzeichnet, dass das Mittel (b)
(b1) mindestens ein Alkylenglycol der Formel (AG-1) enthält, wobei 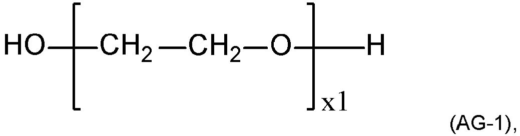
- x1: für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht.

Ein ganz besonders bevorzugtes niedermolekulares Polyethylenglycol ist beispielsweise PEG-8. PEG-8 umfasst im Schnitt 8 Ethylenglycol-Einheiten (x1 = 8), besitzt ein mittleres Molgewicht von 400 g/mol und trägt die CAS-Nummer 25322-68-3. PEG-8 wird alternativ auch als PEG 400 bezeichnet und ist beispielsweise von der Firma APS kommerziell erhältlich.

Weitere gut geeignete niedermolekulare Polyethylenglycole sind beispielsweise PEG-6, PEG-7, PEG-9 und PEG-10.

Ein weiteres gut geeignetes Polyethylenglycol ist beispielsweise PEG-32. PEG-32 umfasst 32 Ethylenglycol-Einheiten (x1 = 32), besitzt ein mittleres Molgewicht von 1500 g/mol und trägt die CAS-Nummer 25322-68-3. PEG-32 wird alternativ auch als PEG 1500 bezeichnet und kann zum Beispiel von der Firma Clariant kommerziell erworben werden.

Weiterhin hat sich auch der Einsatz von hochmolekularen Polyethylenglycolen zur Lösung der erfindungsgemäßen Aufgabenstellung gut geeignet erwiesen.

Hochmolekulare Polyethylenglycole im Sinne der vorliegenden Erfindung können durch die Formel (AG-2) dargestellt werden, wobei die Indexzahl x2 für eine ganze Zahl von 101 bis 10000 steht

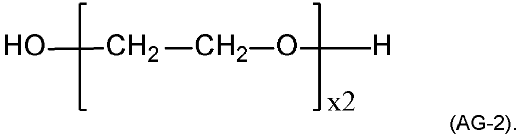

Bei ganz besonders gut geeigneten hochmolekularen Polyethylenglycolen steht x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200.

Im Rahmen einer weiteren ganz besonders bevorzugten Ausführungsform ist ein erfindungsgemäßes Verfahren dadurch gekennzeichnet, dass das Mittel (b)
(b1) mindestens ein Alkylenglycol der Formel (AG-2) enthält, wobei 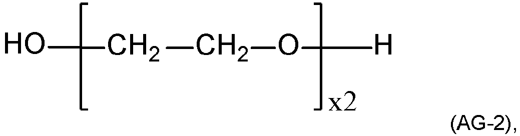
- x2: für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht.

Ein ganz besonders gut geeignetes hochmolekulares Polyethylenglycol ist beispielsweise PEG 6000, welches von der Firma National Starch (China) kommerziell erhalten werden kann. Das Molgewicht von PEG 6000 liegt bei 6000 bis 7500 g/mol, dies entspricht einem x2-Wert von 136 bis 171.

Ein weiteres gut geeignetes Polyethylenglycol ist PEG 12000, das zum Beispiel unter dem Handelsnamen Polyethylene Glycol 12000 S (oder PEG 12000 S) von der Firme CG Chemikalien kommerziell vertrieben wird. Das Molgewicht von PEG 12000 wird mit 10500 bis 15000 g/mol angegeben, entsprechend einem x2-Wert von 238 bis 341.

Ein weiteres gut geeignetes Polyethylenglycol ist auch PEG 20000, welches unter dem Handelsnamen Polyglycol 20000 P bzw. unter dem Alternativnamen PEG-350 von der Firma Clariant käuflich zu erwerblich ist. Für PEG 20000 wird ein Molgewicht von im Schnitt 20000 g/mol angegeben, dies entspricht einem x2-Wert von 454.

Überraschenderweise hat sich herausgestellt, dass Färbemittel, welche sowohl ein niedermolekulares Polyethylenglycol als auch ein hochmolekulares Polyethylenglcyol enthalten, besonders gute anwendungstechnische Eigenschaften besitzen, da diese Mittel sowohl sehr gute Echtheitseigenschaften aufweisen als auch ein im Hinblick auf ihre rheologisches Profil optimiert sind.

Im Rahmen einer weiteren explizit ganz besonders bevorzugten Ausführungsform ist ein erfindungsgemäßes Verfahren daher dadurch gekennzeichnet, dass das Mittel (b)
(b11) mindestens ein erstes Alkylenglycol der Formel (AG-1) enthält, wobei 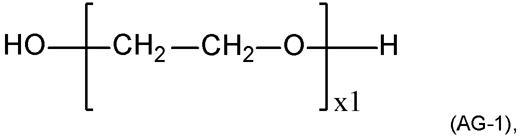
   - x1: für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht, und
(b12) mindestens ein zweites Alkylenglycol der Formel (AG-2) enthält, wobei 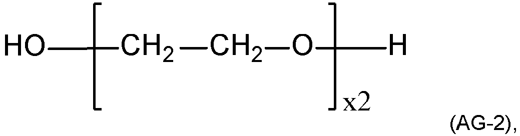
   - x2: für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht.

Zur weiteren Optimierung der anwendungstechnischen Eigenschaften enthält das Mittel (b) das oder die Alkylenglycole (b1) der Formel (AG) bevorzugt in bestimmten Mengenbereichen, die zum Beispiel - bezogen auf das Gesamtgewicht des Mittels - im Bereich von 10,0 bis 99,0 Gew.-%, bevorzugt von 30,0 bis 95,0 Gew.-%, weiter bevorzugt von 40,0 bis 90,0 Gew.-% und ganz besonders bevorzugt von 40,0 bis 80,0 Gew.-% liegen können.

Im Rahmen einer weiteren besonders bevorzugten Ausführungsform ist ein erfindungsgemäßes Verfahren dadurch gekennzeichnet, dass das Mittel (b) - bezogen auf das Gesamtgewicht des Mittels (b) - ein oder mehrere Alkylenglycole (b1) der Formel (AG) in einer Gesamtmenge von 10,0 bis 99,0 Gew.-%, bevorzugt von 30,0 bis 95,0 Gew.-%, weiter bevorzugt von 40,0 bis 90,0 Gew.-% und ganz besonders bevorzugt von 40,0 bis 80,0 Gew.-% enthält.

Im Rahmen einer weiteren besonders bevorzugten Ausführungsform ist ein erfindungsgemäßes Verfahren dadurch gekennzeichnet, dass das Mittel (b) - bezogen auf das Gesamtgewicht des Mittels (b) - ein oder mehrere Alkylenglycole (b1) der Formel (AG-1) und/oder der Formel (AG-2) in einer Gesamtmenge von 10,0 bis 99,0 Gew.-%, bevorzugt von 30,0 bis 95,0 Gew.-%, weiter bevorzugt von 40,0 bis 90,0 Gew.-% und ganz besonders bevorzugt von 40,0 bis 80,0 Gew.-% enthält.

Wassergehalt im Mittel (b)

Bei dem Mittel (b) kann es sich auch um ein wasserbasiertes Mittel handelt, dass zusätzlich oder anstatt der Alkylenglycole Wasser enthält. Bevorzugt besitzt das Mittel (b) einen geringen bis mittleren Wasseranteil. Es hat sich herausgestellt, dass besonders die Mittel gut geeignet sind, die - bezogen auf das Gesamtgewicht des Mittels - 0,1 bis 50,0 Gew.-%, bevorzugt 0,5 bis 35,0 Gew.-%, weiter bevorzugt 1,0 bis 20,0 Gew.-% und besonders bevorzugt 1,5 bis 15,0 Gew.-% Wasser enthalten.

In einer weiteren explizit Ausführungsform ist ein erfindungsgemäßes Verfahren dadurch gekennzeichnet, dass das Mittel (b) - bezogen auf das Gesamtgewicht des Mittels (b) - 0,1 bis 50,0 Gew.-%, bevorzugt 0,5 bis 35,0 Gew.-%, weiter bevorzugt 1,0 bis 20,0 Gew.-% und besonders bevorzugt 1,5 bis 15,0 Gew.-% Wasser enthält.

### Einsatz von Pigmenten im Verfahren zur Behandlung von Keratinfasern

Prinzipiell ist das erfindungsgemäße Verfahren für verschiedene Verfahren zur Behandlung von Keratinfasern bzw. Haaren geeignet. Dies können all die Verfahren sein, bei denen eine gleichmäßige Auftragung des Aminosilikons (a1) von besonderer Bedeutung ist, wie beispielsweise einer Färbung, einer Haarglättung oder einer Dauerwelle. Bei einer auf Pigmenten basierenden Haarfärbung resultiert die ungleichmäßige Beaufschlagung der Haare mit Aminosilikon in einem ungleichmäßigen Farbergebnis und wird für den Anwender daher besonders stark sichtbar. Aus diesem Grund ist es explizit ganz besonders bevorzugt, wenn es sich bei dem erfindungsgemäßen Verfahren um ein pigmentbasiertes Färbeverfahren handelt.

Bei Anwendung der Mittel (a) und (b) in eine Färbeverfahren haben sich verschiedene Ausführungsformen als besonders gut geeignet heraus gestellt. Im Rahmen dieser Ausführungsform können ein oder mehrere Pigmente direkt in das Mittel (b) eingearbeitet werden, so dass das Mittel (b) auch als Färbemittel bezeichnet werden kann.

Im Rahmen einer weiteren besonders bevorzugten Ausführungsform ist ein erfindungsgemäßes Verfahren dadurch gekennzeichnet, dass es ein Verfahren zum Färben von keratinischem Material, insbesondere menschlichen Haaren, ist und dass das Mittel (b) enthält:
(b2) mindestens ein Pigment.

Das anwendungsberiete Färbemittel wird im Rahmen dieser Ausführungsform durch das Vermischen der Mittel (a) und (b) hergestellt.

Ganz besonders bevorzugt ist damit (entsprechend umnummeriert) ein Verfahren zur Färbung von keratinischen Fasern, insbesondere menschlichen Haaren, umfassend die folgenden Schritte:
(1) Bereitstellung eines Mittels (a), wobei das Mittel (a) enthält:
   (a1) mindestens ein aminofunktionalisiertes Silikonpolymer, und
   (a2) mindestens ein bei 20 °C flüssiges kosmetisches Öl, das von (a1) verschieden ist,
(2) Bereitstellung eines Mittels (b), wobei das Mittel (b) enthält:
   (b1) Wasser und/oder mindestens Alkylenglycol der Formel (AG) 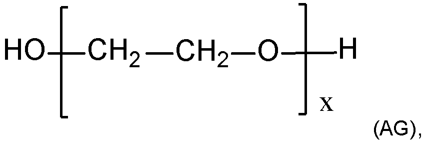 wobei
      - x: für eine ganze Zahl von 1 bis 10000 steht, und
   (b2) mindestens ein Pigment,
(3) Herstellung einer Anwendungsmischung durch Vermischen der Mittel (a) und (b),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) gegebenenfalls Ausspülen der Anwendungsmischung.

Bestimmte Pigmente wie insbesondere Metallpigmente oder Glanzpigmente haben sich bisweilen als instabil in wasserhaltiger Lösung erwiesen. Da das Mittel (b) wie zuvor beschrieben bevorzugt einen gewissen Wasseranteil hat, können empfindliche Pigmente in diesem Mittel korrodieren. Zur Vermeidung dieser Inkompatitilitäten können die Pigmente daher auch in einem weiteren separaten Mittel (c) konfektioniert werden.

Im Rahmen einer weiteren besonders bevorzugten Ausführungsform ist ein erfindungsgemäßes Verfahren dadurch gekennzeichnet, dass es ein Verfahren zum Färben von keratinischem Material, insbesondere menschlichen Haaren, ist, und weiterhin gekennzeichnet durch die
(3) Bereitstellung eines Mittels (c), wobei das Mittel (c) enthält:
   (c1) mindestens ein Pigment, und die
(4) Herstellung einer Anwendungsmischung durch Vermischen der Mittel (a) und (b) und (c).

Das anwendungsberiete Färbemittel wird im Rahmen dieser Ausführungsform durch das Vermischen der Mittel (a) und (b) und (c) hergestellt.

Ganz besonders bevorzugt ist damit ein Verfahren zur Färbung von keratinischen Fasern, insbesondere menschlichen Haaren, umfassend die folgenden Schritte:
(1) Bereitstellung eines Mittels (a), wobei das Mittel (a) enthält:
   (a1) mindestens ein aminofunktionalisiertes Silikonpolymer, und
   (a2) mindestens ein bei 20 °C flüssiges kosmetisches Öl, das von (a1) verschieden ist,
(2) Bereitstellung eines Mittels (b), wobei das Mittel (b) enthält:
   (b1) Wasser und/oder mindestens Alkylenglycol der Formel (AG) 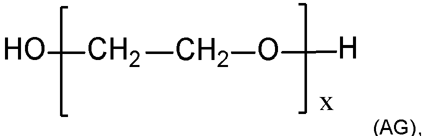 wobei
   - x: für eine ganze Zahl von 1 bis 10000 steht,
(3) Bereitstellung eines Mittels (c), wobei das Mittel (c) enthält:
   (c1) mindestens ein Pigment,
(4) Herstellung einer Anwendungsmischung durch Vermischen der Mittel (a) und (b) und (c),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) gegebenenfalls Ausspülen der Anwendungsmischung.

Bei Einsatz von Pigmentmischungen ist es ebenfalls möglich, wenn ein erstes Pigment oder eine erste Pigmentmischung im Mittel (b) eingesetzt wird und ein zweites Pigment oder eine zweite Pigmentmischung im Mittel (c) zur Anwendung kommt.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel (b) dadurch gekennzeichnet, dass es mindestens eine farbgebende Verbindung (b3) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Mittel (b) und/oder Mittel (c) mindestens ein Pigment aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass das Mittel (b) und/oder das Mittel (c) mindestens ein Pigment aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Zur Färbung der Keratinfasern können auch Pigmente mit einer bestimmten Formgebung eingesetzt worden sein. Beispielsweise kann ein Pigment auf Basis eines lamellaren und/oder eines lentikularen Substratplättchens eingesetzt werden. Weiterhin ist auch die Färbung auf Basis eines Substratplättchens möglich, welches ein Vakuum metallisiertes Pigment umfasst.

Im Rahmen einer weiteren bevorzugten Ausführungsform ist ein erfindungsgemäßes Verfahren dadurch gekennzeichnet, dass das Mittel (b) und /oder das Mittel (c) mindestens ein Pigment enthält, das ausgewählt ist aus der Gruppe der Pigmente auf Basis eines lamellaren Substratplättchens, der Pigmente auf Basis eines lentikularen Substratplättchens und der Vakuum metallisierten Pigmente.

Die Substratplättchen dieses Typs weisen eine durchschnittliche Dicke von höchstens 50 nm, vorzugsweise weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Substratplättchen sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf.

Durch die geringe Dicke der Substratplättchen weist das Pigment ein besonders hohes Deckvermögen auf.

Die Substratplättchen sind bevorzugt monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können. Die Substratplättchen sind vorzugsweise homogen aufgebaut, d.h. dass innerhalb der Plättchen kein Konzentrationsgradient auftritt. Insbesondere sind die Substratplättchen nicht schichtartig aufgebaut und weisen keine darin verteilten Teilchen oder Partikel auf.

Die Größe des Substratplättchens kann auf den jeweiligen Anwendungszweck, insbesondere dem gewünschten Effekt auf dem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750, beträgt. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen können aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus Metall(legierung)en aufgebaut.

Als Metall kommt jedes für metallische Glanzpigmente geeignete Metall in Betracht. Derartige Metalle sind unter anderem Eisen und Stahl, sowie alle luft- und wasserbeständigen (Halb)metalle wie beispielsweise Platin, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Lamellare Substratplättchen zeichnen sich durch einen unregelmäßig strukturierten Rand aus und werden aufgrund ihres Erscheinungsbildes auch als "cornflakes" bezeichnet.

Aufgrund ihrer unregelmäßigen Struktur erzeugen Pigmente auf der Basis von lamellaren Substratplättchen einen hohen Anteil an Streulicht. Außerdem decken die Pigmente auf der Basis von lamellaren Substratplättchen die vorhandene Farbe eines keratinischen Materials nicht vollständig ab und es können beispielsweise Effekte analog zu einer natürlichen Ergrauung erzielt werden.

Lentikulare (= linsenförmige) Substratplättchen weisen einen im Wesentlichen regelmäßigen runden Rand auf und werden aufgrund ihres Erscheinungsbildes auch als "silverdollars" bezeichnet. Aufgrund ihrer regelmäßigen Struktur überwiegt bei Pigmenten auf Basis von lentikularen Substratplättchen der Anteil des reflektierten Lichts.

Vakuum metallisierte Pigmente (*vacuum metallized pigments,* VMP) können beispielsweise durch das Freisetzen von Metallen, Metalllegierungen oder Metalloxiden von entsprechend beschichteten Folien gewonnen werden. Sie zeichnen sich durch eine besonders geringe Dicke der Substratplättchen im Bereich von 5 bis 50 nm und durch eine besonders glatte Oberfläche mit erhöhter Reflektivität aus. Substratplättchen, welche ein im Vakuum metallisiertes Pigment umfassen, werden im Rahmen dieser Anmeldung auch als VMP-Substratplättchen bezeichnet. VMP-Substratplättchen aus Aluminium können beispielsweise durch Freisetzen von Aluminium von metallisierten Folien gewonnen werden.

Die Substratplättchen aus Metall oder Metalllegierung können passiviert sein, beispielsweise durch Eloxieren (Oxidschicht) oder Chromatieren.

Unbeschichtete lamellare, lentikulare und/oder VPM-Substratplättchen, insbesondere solche aus Metall oder Metalllegierung, reflektieren das einfallende Licht in hohem Maße und erzeugen einen Hell-Dunkel-Flop. Diese haben sich zum Einsatz im Färbemittel als besonders bevorzugt erwiesen.

Geeignete Pigmente auf Basis eines lamellaren Substratplättchens umfassen beispielsweise die Pigmente der Reihe VISIONAIRE von Eckart.

Pigmente auf Basis eines lentikularen Substratplättchens sind beispielsweise unter der Bezeichnung Alegrace^{®} Gorgeous von der Firma Schlenk Metallic Pigments GmbH erhältlich.

Pigmente auf Basis eines Substratplättchens, welches ein Vakuum metallisiertes Pigment umfasst, sind beispielsweise unter der Bezeichnung Alegrace^{®} Marvelous oder Alegrace^{®} Aurous von der Firma Schlenk Metallic Pigments GmbH erhältlich.

Aufgrund ihrer ausgezeichneten Licht- und Temperaturbeständigkeit ist die Verwendung der zuvor genannten Pigmente in dem erfindungsgemäßen Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispiel

Das oder die Pigmente werden ganz besonders bevorzugt in bestimmten Mengenbereichen im Mittel (b) und/oder im Mittel (c) eingesetzt. Besonders gute Ergebnisse wurden erhalten, wenn das Mittel (b) und/oder das Mittel (c) - bezogen auf das Gesamtgewicht des jeweiligen Mittels - ein odere mehrere Pigmente in einer Gesamtmenge von 0,05 bis 10,0 Gew.-%, bevorzugt 0,1 bis 7,0 Gew.-%, weiter bevorzugt von 0,2 bis 5,0 Gew.-% und ganz besonders bevorzugt von 0,3 bis 3,0 Gew.-% enthielt.

In einer weiteren ganz besonders bevorzugten Ausführungsform ist ein erfindungsgemäßes Verfahren dadurch gekennzeichnet, dass das Mittel (b) und/oder das Mittel (c) - bezogen auf das Gesamtgewicht des jeweiligen Mittels - ein odere mehrere Pigmente in einer Gesamtmenge von 0,05 bis 10,0 Gew.-%, bevorzugt 0,1 bis 7,0 Gew.-%, weiter bevorzugt von 0,2 bis 5,0 Gew.-% und ganz besonders bevorzugt von 0,3 bis 3,0 Gew.-% enthält.

### Anlagerungsprodukte von C₁-C₆-Alkylenoxid(en) an die Ester aus C₁₂-C₃₀-Fettsäuren und aromatischen C₁-C₁₂-Alkoholen (a5)

Zur weiteren Verbesserung der Echtheitseigenschaften wie Reibechtheit und Waschechtheit können die erfindungsgemäßen Mittel als optionalen Bestandteil zusätzlich mindestens ein Anlagerungsprodukt von C₁-C₆-Alkylenoxid(en) an die Ester aus C₁₂-C₃₀-Fettsäuren und aromatischen C₁-C₁₂-Alkoholen enthalten, die unter die Verfindungen der allgemeinen Formel (AFE-I) fallen 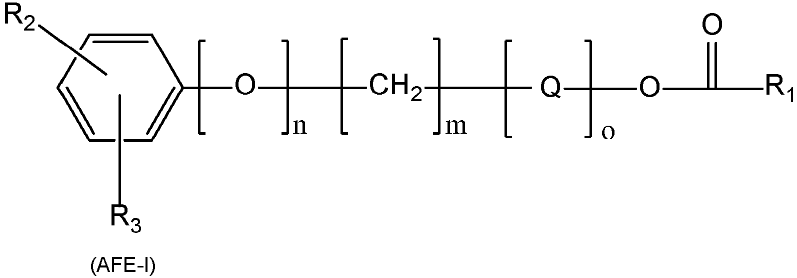 wobei
- R1: für eine gesättigte oder ungesättigte C₁₁-C₂₉-Alkylgruppe steht
- R2, R3: unabhängig voneinander für ein Wasserstoffatom, eine C₁-C₆-Alkylgruppe, eine Hydroxygruppe oder eine C₁-C₆-Alkoxygruppe stehen,
- n: für die Zahl 0 oder 1 steht, für eine ganze Zahl von 0 bis 6 steht,
- m o: für eine ganze Zahl von 1 bis 60 steht, und
- Q: für eine Struktureinheit -O-CH₂-CH₂- , -O-CH(CH₃)-CH₂- oder -O-CH₂-CH(CH₃)- steht,
wobei die Maßgabe besteht, dass, wenn m gleich 0 ist, auch n gleich 0 ist.

Im Rahmen einer weiteren besonders bevorzugten Ausführungsform ist ein erfindungsgemäßes Verfahren dadurch gekennzeichnet, dass das Mittel (b) enthält:
(b3) mindestens einen alkoxylierten Fettsäureester der allgemeinen Formel (AFE-I) 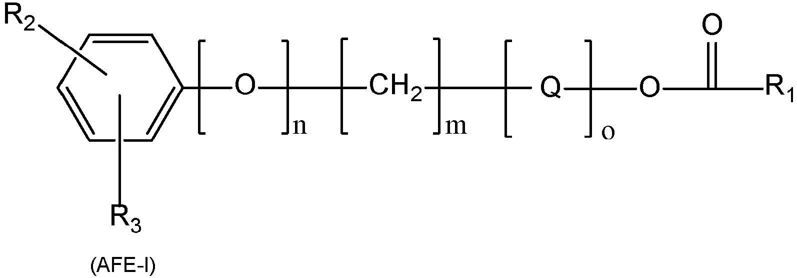 wobei
- R1: für eine gesättigte oder ungesättigte C₁₁-C₂₉-Alkylgruppe steht,
- R2, R3: unabhängig voneinander für ein Wasserstoffatom, eine C₁-C₆-Alkylgruppe, eine Hydroxygruppe oder eine C₁-C₆-Alkoxygruppe stehen,
- n: für die Zahl 0 oder 1 steht,
- m: für eine ganze Zahl von 0 bis 6 steht,
- o: für eine ganze Zahl von 1 bis 60 steht, und
- Q: für eine Struktureinheit -O-CH₂-CH₂- , -O-CH(CH₃)-CH₂- oder -O-CH₂-CH(CH₃)- steht,
wobei die Maßgabe besteht, dass, wenn m gleich 0 ist, auch n gleich 0 ist.

Der Rest R1 steht für eine gesättigte oder ungesättigte C₁₁-C₂₉-Alkylgruppe. Eine ungesättigte C₁₁-C₂₃-Alkylgruppe kann eine oder mehrere Doppelbindungen umfassen und wird alternativ auch als ungesättigte C₁₁-C₂₃-Alkenylgruppe bezeichnet. Die gesättigte oder ungesättigte C₁₁-C₂₉-Alkylgruppe kann linear oder verzweigt sein.

Bevorzugt steht R1 für eine lineare, gesättigte oder ungesättigte C₁₁-C₂₃-Alkylgruppe.

Die Reste R2 und R3 stehen voneinander für ein Wasserstoffatom, eine C₁-C₆-Alkylgruppe, eine Hydroxygruppe oder eine C₁-C₆-Alkoxygruppe. Ganz besonders bevorzugt stehen die Reste R2 und R3 beide für ein Wasserstoffatom.

Die Index-Zahl n steht für die Zahl 0 oder 1. Bevorzugt steht n für die Zahl 0.

Die Index-Zahl m steht für eine ganze Zahl von 0 bis 6. Bevorzugt steht m für die Zahl 1.

Die Index-Zahl o steht für eine ganze Zahl von 1 bis 60. Bevorzugt steht o für eine ganze Zahl von 1 bis 30, weiter bevorzugt von 1 bis 20, noch weiter bevorzugt von 1 bis 10 und ganz besonders bevorzugt von 1 bis 5.

Der Rest Q steht für eine Struktureinheit -O-CH₂-CH₂- , -O-CH(CH₃)-CH₂- oder -O-CH₂-CH(CH₃)-steht. Besonders bevorzugt steht Q für eine Struktureinheit -O-CH(CH₃)-CH₂- oder -O-CH₂-CH(CH₃)-.

Wenn o für eine Zahl von größer als 1 steht, sind in den Verbindungen der Formel (AFE-I) (bzw. auch der Formel (AFE-II) mehrere Struktureinheiten Q enthalten, in diesem Fall kann jede Struktureinheit Q von den anderen Struktureinheiten Q unabhängig gewählt werden.

Im Rahmen einer weiteren ganz besonders bevorzugten Ausführungsform ist ein erfindungsgemäßes Mittel dadurch gekennzeichnet, dass es
(b3) mindestens einen alkoxylierten Fettsäureester der allgemeinen Formel (AFE-I) enthält 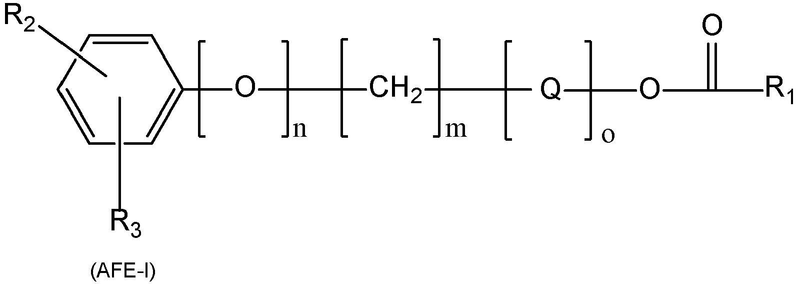 wobei
- R1: für eine gesättigte oder ungesättigte C₁₁-C₂₉-Alkylgruppe steht
- R2, R3: beide für ein Wasserstoffatom stehen,
- n: für die Zahl 0 steht,
- m: für die Zahl 1 steht,
- o: für eine ganze Zahl von 1 bis 30, bevorzugt von 1 bis 20, weiter bevorzugt von 1 bis 10 und ganz besonders bevorzugt von 1 bis 5 steht, und
- Q: für eine Struktureinheit -O-CH(CH₃)-CH₂- oder -O-CH₂-CH(CH₃)- steht.

Bei einer explizit ganz besonders gut geeigneten Verbindung dieses Typs handelt es sich um PPG-3 Benzyl Ether Myristate, das alternativ auch als α-(1-Oxotetradecyl)-ω-(phenylmethoxy) Poly[oxy(methyl-1,2-ethanediyl)] bezeichnet wird und die CAS-Nummer 642443-86-5 trägt.

PPG-3 Benzyl Ether Myristate kann zum Beispiel unter dem Handelsnamen Crodamol STS von der Firma Croda käuflich erworben werden.

Im Rahmen einer weiteren ganz besonders bevorzugten Ausführungsform ist ein erfindungsgemäßes Verfahren dadurch gekennzeichnet, dass das Mittel (b) PPG-3 Benzyl Ether Myristate enthält.

Die alkoxylierten Fettsäureester (b3) werden besonders bevorzugt in bestimmten Mengenbereichen im erfindungsgemäßen Mittel eingesetzt. Besonders gute Ergebnisse wurden erhalten, wenn das Mittel (b) - bezogen auf das Gesamtgewicht des Mittels (b) - ein oder mehrere alkoxylierte Fettsäureester (b3) in einer Gesamtmenge von 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 8,0 Gew.-% und ganz besonders bevorzugt von 1,0 bis 5,0 Gew.-% enthält.

### Anlagerungsprodukte von C₁-C₆-Alkylenoxid(en) an aliphatische C₁-C₂₄-Alkanole

Zur weiteren Verbesserung der Echtheitseigenschaften wie Reibechtheit und Waschechtheit können die erfindungsgemäßen Mittel (b) als optionalen Bestandteil zusätzlich mindestens ein Anlagerungsprodukt von C₁-C₆-Alkylenoxiden an C1-C24-Alkanole enthalten.

Im Rahmen einer weiteren ganz besonders bevorzugten Aufführungsform ist ein erfindungsgemäßes Verfahren daher weiterhin dadurch gekennzeichnet dass das Mittel (b)
(b4) mindestens ein Anlagerungsprodukt von C₁-C₆-Alkylenoxid(en) an aliphatische C₁-C₂₄-Alkanole enthält.

Die weiteren optional enthaltenen Bestandteile (b4) können zusätzlich zu den Inhaltsstoffen der Gruppe (b3) eingesetzt werden. Die weiteren optional enthaltenen Bestandteile (b4) können aber auch anstatt der Inhaltsstoffe der Gruppe (b3) eingesetzt werden.

Erfindungsgemäß geeignete C₁-C₆-Alkylenoxide und deren bevorzugte und besonders bevorzugte Vertreter wurden bereits in den vorangehenden Abschnitten definiert.

Bei den C₁-C₂₄-Alkanolen handelt es sich erfindungsgemäß um Verbindungen mit 1 bis 14 Kohlenstoffatomen und einer Hydroxygruppe. Beispielhaft genannt werden können Methanol, Ethanol, 1-Propanol, 2-Propanol, 1-Butanol, 2-Butanol, 1-Pentanol, 2-Pentanol, 3-Pentanol, 1-Hexanol, 2-Hexanol, 3-Hexanol, 1-Heptanol, 2-Heptanol, 3-Heptanol, 4-Heptanol, 1-Octanol, 2-Octanol, 3-Octanol, 4-Octanol, 1-Nonanol, 2-Nonanol, 3-Nonanol, 4-Nonanol, 5-Nonanol, 1-Decanol, 2-Decanol, 3-Decanol, 4-Decanol, 5-Decanol, 1-Undecanol, 2-Undecanol, 3-Undecanol, 4-Undecanol, 5-Undecanol, 6-Undecanol, 1-Dodecanol, 2-Dodecanol, 3-Dodecanol, 4-Dodecanol, 5-Dodecanol, 6-Dodecanol, 1-Tridecanol, 2-Tridecanol, 3-Tridecanol, 4-Tridecanol, 5-Tridecanol, 6-Tridecanol, 7-Tridecanol, 1-Tetradecanol, 2-Tetradecanol, 3-Tetradecanol, 4-Tetradecanol, 5-Tetradecanol, 6-Tetradecanol, 7-Tetradecanol, 1-Pentadecanol, 2-Pentadecanol, 3-Pentadecanol, 4-Pentadecanol, 5-Pentadecanol, 6-Pentadecanol, 7-Pentadecanol, 8-Pentadecanol, 1-Hexadecanol, 2-Hexadecanol, 3-Hexadecanol, 4-Hexadecanol, 5-Hexadecanol, 6-Hexadecanol, 7-Hexadecanol, 8-Hexadecanol, 1-Heptadecanol, 2-Heptadecanol, 3-Heptadecanol, 4-Heptadecanol, 5-Heptadecanol, 6-Heptadecanol, 7-Heptadecanol, 8-Heptadecanol, 1-Octadecanol, 2-Octadecanol, 3-Octadecanol, 4-Octadecanol, 5-Octadecanol, 6-Octadecanol, 7-Octadecanol, 8-Octadecanol und 9-Octadecanol.

Besonders gut geeignete Anlagerungsprodukte von C₁-C₆-Alkylenoxid(en) an aliphatische C₁-C₂₄-Alkanole (b4) sind die Verbindungen der allgemeinen Formel (AA-I) 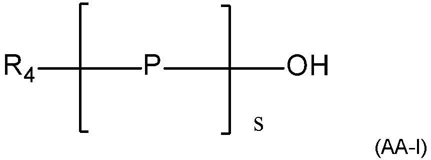 worin
- R4: für eine gesättigte oder ungesättigte C₁-C₂₄-Alkylgruppe steht und
- P: für eine Struktureinheit -O-CH₂-CH₂- , -O-CH(CH₃)-CH₂- oder -O-CH₂-CH(CH₃)-steht, und
- s: für eine ganze Zahl von 1 bis 60 steht.

Der Rest R4 steht für eine gesättigte oder ungesättigte, C₁-C₂₄-Alkylgruppe. Ungesättigt können die Reste R4 ab einer Kohlenstoffanzahl von mindestens 2 C-Atomen sein. Eine ungesättigte C₂-C₂₄-Alkylgruppe kann eine oder mehrere Doppelbindungen umfassen und wird alternativ auch als C₂-C₂₄-Alkenylgruppe bezeichnet. Die gesättigte oder ungesättigte C₁-C₂₄-Alkylgruppe kann linear oder verzweigt sein.

Besonders bevorzugt steht der Rest R4 für eine gesättigte, unverzweigte C₁-C₁₂-Alkylgruppe. Ganz besonders bevorzugt steht der Rest R4 für eine gesättigte, unverzweigte C₁-C₆-Alkylgruppe.

In den Verbindungen der Formel (AA-I) steht P für eine Struktureinheit -O-CH₂-CH₂- , -O-CH(CH₃)-CH₂- oder -O-CH₂-CH(CH₃)- steht. Die Anzahl der in den Verbindungen der Formel (AA-I) enthaltenen Struktureinheiten ergibt sich durch die Indexzahl s. Hierbei sind die Struktureinheiten P so ausgerichtet, dass sich das Sauerstoffatom in jeder Gruppierung -O-CH₂-CH₂-, -O-CH(CH₃)-CH₂-und -O-CH₂-CH(CH₃)- zum Alkylrest R4 benachbart befindet, und die jeweilige Einheit -CH2- bzw. - CH(CH3)- an die Hydroxy-Gruppe -OH angrenzt.

### Wenn s für eine Zahl von größer als 1 steht, sind in den Verbindungen der Formel (AA-I) mehrere Struktureinheiten P enthalten. In diesem Fall kann jede Struktureinheit P von den anderen Struktureinheiten P unabhängig gewählt werden.

Die Indexzahl s steht für eine ganze Zahl von 1 bis 60, bevorzugt für eine ganze Zahl von 1 bis 40, weiter bevorzugt für eine ganze Zahl von 10 bis 30 und ganz besonders bevorzugt für eine ganze Zahl von 10 bis 20.

Im Rahmen einer weiteren ganz besonders bevorzugten Aufführungsform ist ein erfindungsgemäßes Verfahren dadurch gekennzeichnet, dass das Mittel (b) enthält:
(b4) mindestens ein Anlagerungsprodukt von C₁-C₆-Alkylenoxid(en) an aliphatische C₁-C₂₄-Alkanole der Formel (AA-I), 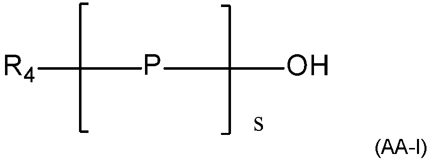 worin
- R4: für eine gesättigte oder ungesättigte C₁-C₂₄-Alkylgruppe, bevorzugt für eine C₁-C₁₂-Alkylgruppe, besonders bevorzugt für eine C₁-C₆-Alkylgruppe, steht, und
- P: für eine Struktureinheit -O-CH₂-CH₂- , -O-CH(CH₃)-CH₂- oder -O-CH₂-CH(CH₃)-steht, und
- s: für eine ganze Zahl von 1 bis 60, bevorzugt für eine ganze Zahl von 1 bis 40, weiter bevorzugt für eine ganze Zahl von 10 bis 30 und ganz besonders bevorzugt für eine ganze Zahl von 10 bis 20, steht.

Bei einem ganz besonders gut geeigneten Anlagerungsprodukt von C₁-C₆-Alkylenoxid(en) an aliphatische C₁-C₂₄-Alkanole der Formel (AA-I) handelt es sich um Propylene Glycol Monobutylether, das auch als PPG-14 Butyl ether bezeichnet wird und die CAS-Nummer 9003-13-8. Trägt. PPG-14 Butyl ether kann kommerziell unter dem Handelsnamen Ucon Fluid AP von der Firma Dow erworben werden.

Im Rahmen einer weiteren ganz besonders bevorzugten Aufführungsform ist ein erfindungsgemäßes Verfahren daher weiterhin dadurch gekennzeichnet dass das Mittel (b) PPG-14 Butyl ether enthält.

Die alkoxylierten Alkanole (b4) werden besonders bevorzugt in bestimmten Mengenbereichen im erfindungsgemäßen Mittel eingesetzt.

Besonders gute Ergebnisse wurden erhalten, wenn das Mittel (b) - bezogen auf das Gesamt-gewicht des Mittels (b) - ein oder mehrere alkoxylierten Alkanole (b4) in einer Gesamtmenge von 0,1 bis 20,0 Gew.-%, bevorzugt 0,2 bis 15,0 Gew.-%, weiter bevorzugt von 0,3 bis 10,0 Gew.-%, noch weiter bevorzugt von 0,4 bis 5,0 Gew.-% und ganz besonders bevorzugt von 0,5 bis 3,0 Gew.-% enthielt.

### weitere optionale Inhalttstoffe in den Mitteln (a), (b) und (c)

Zusätzlich zu den bereits beschriebenen erfindungswesentlichen und optionalen Bestandteilen können die Mittel (a) und/oder (b) und/oder (c) auch noch weitere optionale Inhaltsstoffe enthalten.

Die Mittel können ferner auch noch weitere Wirk-, Hilfs- und Zusatzstoffe enthalten, wie beispielsweise Tenside, filmbildende Polymere, Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Herstellung der Anwendungsmischung durch Vermischen der Mittel (a) und (b) und ggf. (c)

In Schritt (4) des erfindungsgemäßen Verfahrens wird durch Vermischen der Mittel (a) und (b) eine anwendungsbereite Mischung hergestellt. Wird ein separat konfektioniert ein Pigment in einem dritten Mittel (c) eingesetzt, so wird in Schritt (4) des Verfahrens durch Vermischen der Mittel (a) und (b) und (c) eine anwendungsbereites Färbemittel hergestellt.

Prinzipiell können verschiedene Mengen des Mittels (a) mit dem Mittel (b) vermischt werden, so sind prinzipell Mischungsverhältniss (a)/(b) von 1:400 bis 400:1 denkbar.

Besonders bevorzugt ist es, wenn die Anwendungsmischung durch Vermischen der Mittel (a) und (b) im Gewichtsmengenverhältnis (a)/(b) von 1:1 bis 1:100, bevorzugt von 1:3 bis 1:50, weiter bevorzugt von 1:5 bis 1:40, noch weiter bevorzugt von 1:7 bis 1:30 hergestellt wird.

Bei einem Mengenverhältnis (a)/(b) von 1:120 können beispielsweise 1 g Mittel (a) mit 120 g Mittel (b) vermischt werden.

Bei einem Mengenverhältnis (a)/(b) von 1:100 können beispielsweise 1 g Mittel (a) mit 100 g Mittel (b) vermischt werden.

Bei einem Mengenverhältnis (a)/(b) von 1:15 können beispielsweise 10 g Mittel (a) mit 150 g Mittel (b) vermischt werden.

Bei einem Mengenverhältnis (a)/(b) von 1:25 können beispielsweise 4 g Mittel (a) mit 100 g Mittel (b) vermischt werden.

Im Rahmen einer weiteren bevorzugten Ausführungsform ist ein erfindungsgemäßes Verfahren gekennzeichnet durch die
(3) Herstellung einer Anwendungsmischung durch Vermischen der Mittel (a) und (b) und gegebenenfalls (c), wobei die Mittel (a) und (b) in einem Gewichtsverhältnis (a)/(b) von 1:1 bis 1:100, bevorzugt von 1:3 bis 1:50, weiter bevorzugt von 1:5 bis 1:40, noch weiter bevorzugt von 1:7 bis 1:30 und ganz besonders bevorzugt von 1:9 bis 1:28 miteinander vermischt werden.

### Applizieren der Anwendungsmischung

In Schritt (5) des erfindungsgemäßen Verfahrens wird die in Schritt (4) hergestellte Anwendungsmischung auf die Keratinfasern bzw. Haare appliziert.

Bevorzugt wird die Anwendungsmischung innerhalb eines Zeitraums von 1 bis 120 Minuten, bevorzugt von 1 bis 60 Minuten, weiter bevorzugt 1 bis 30 Minuten und ganz besonders bevorzugt von 1 bis 15 Minuten nach ihrer Herstellung in Schritt (4) auf das Keratinmaterial (bzw. auf die Haare) appliziert.

In einer weiteren bevorzugten Ausführungsform ist ein erfindungsgemäßes Verfahren gekennzeichnet durch das
(5) Applizieren Anwendungsmischung auf dem keratinischen Material innerhalb eines Zeitraums von 1 bis 120 Minuten, bevorzugt von 1 bis 60 Minuten, weiter bevorzugt 1 bis 30 Minuten und ganz besonders bevorzugt von 1 bis 15 Minuten nach ihrer Herstellung in Schritt (4).

### Einwirken der Anwendunqsmischunq auf das Keratinmaterial

In Schritt (6) des erfindungsgemäßen Verfahrens wird die Anwendungsmischung nach ihrer Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das
(6) Einwirken der in Schritt (5) applizierten Anwendungsmischung auf dem keratinischen Material für einen Zeitraum von 30 Sekunden bis 15 Minuten, bevorzugt von 30 Sekunden bis 10 Minuten, und besonders bevorzugt von 1 bis 5 Minuten

### Ausspülen der Anwendungsmischung

Im Anschluss an das Einwirken der Anwendungsmischung auf das Keratinmaterial wird diese schließlich in Schritt (7) mit Wasser ausgespült.

Hierbei kann die Awendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners in Schritt (8) ist prinzipiell denkbar.

### Abfolge der Verfahrensschritte

Das erfindungsgemäße Verfahren umfasst die Schritte (1) bis (7) (bzw. (1) bis (6), wenn kein drittes Mittel (c) zur Anwendung kommen soll).

In Schritt (1) wird das Mittel (a) bereitgestellt, Schritt (2) umfasst die Bereitstellung des Mittels (b). In Schritt (3) wird das optionale Mittel (c ) bereitgestellt.

Diese zwei bzw. drei Schritte müssen zeitlich nicht zwingend nacheinander erfolgen, sondern können auch gleichzeitig ablaufen.

So kann Schritt (1) vor Schritt (2) erfolgen, die Schritte (1) und (2) können gleichzeitig ablaufen oder aber Schritt (2) erfolgt vor Schritt (1). Schritt (3) kann vor oder nach Schritt (1) bzw. (2) erfolgen. Auch die Schritte (1), (2) und (3) kölnnen gleichzeitig ablaufen.

Werden dem Anwender beispielsweise die Mittel (a) und (b) und ggf. (c) in einer Mehrkomponenten-Verpackungseinheit zur Vergügung gestellt, so werden die Mittel gleichzeitig bereitgestellt, und es bleibt dem Anwender überlassen, welches Mittel er zuerst aus der Verpackung entnimmt.

Die Herstellung einer Anwendungsmischung durch Vermischen der Mittel (a) und (b) und ggf. (c) in Schritt (4) kann nur nach Bereitstellung der Mittel (a) und (b) und ggf. (c) erfolgen. Das Applizieren der Anwendungsmischung in Schritt (5) kann erst nach deren Herstellung in Schritt (4) erfolgen. Analog kann das Einwirken der Anwendungsmischung in Schritt (6) erst nach deren Aufbringen auf das Keratinmaterial ergolgen, und das Ausspülen der Anwendungmischung in Schritt (7) erfolgt nach deren Einwirken in Schritt (6).

### Mehrkomponenten-Verpackungseinheit

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zur Behandlung von keratinischem Material, insbesondere menschlichen Haaren, umfassend getrennt voneinander konfektioniert
- einen ersten Container mit einem Mittel (a), wobei das Mittel (a) enthält:
   (a1) mindestens ein aminofunktionalisiertes Silikonpolymer, und
   (a2) mindestens ein bei 20 °C flüssiges kosmetisches Öl, das von (a1) verschieden ist, und
- einen zweiten Container mit einem Mittel (b), wobei das Mittel (b) enthält:
   (b1) Wasser und/oder mindestens Alkylenglycol der Formel (AG) 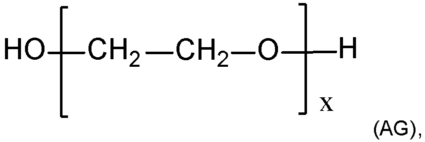 wobei
   - x: für eine ganze Zahl von 1 bis 10000 steht, und
- gegebenenfalls einen dritten Container mit einem Mittel (c), wobei das Mittel (c) enthält:
   (c1) mindestens ein Pigment,
   wobei die Mittel (a), (b) und (c) und die Bestandteile (a1), (a2), (b1) und (c1) bei der Beschreibung des ersten Erfindungsgegenstands im Detail offenbart sind.

Betreffend die weiteren bevorzugten Ausführungsformen der erfindungsgemäßen Mehrkomponenten-Verpackungseinheit gilt *mutatis mutantis* das zum erfindungsgemäßen Verfahren gesagte.

### Beispiele

### 1. Formulierungen

Es wurden die folgenden Formulierungen hergestellt:

| Mittel (a) | (aV) Vergleich | (aE) Erfindung |
|---|---|---|
| Dow Corning 2-8566 (Siloxanes and Silicones, 3-[(2-Aminoethyl)amino]-2-methylpropyl Me, Di-Me-Siloxane] | 0,75 g | 0,75 g |
| Hexamethyldisiloxane (Viskosität 0,65 cSt) | --- | 6,75 g |
| Gesamtmenge Mittel (a) | 0,75 g | 7,5 g |

| Mittel (b) | (b) |
|---|---|
| Wasser | 10,0 g |
| PPG-3 Benzyl Ether Myristate | 1,5 g |
| Ucon Fluid AP | 1,0 g |
| Phenoxyethanol | 0,2 g |
| 4-Hydroxyacetophenon | 0,2 g |
| Unipure Red LC 3079 (Pigment) | 1,0 g |
| Polyethylenglycol (Molgewicht 6000 g/mol) | 10,0 g |
| Polyethylenglycol (Molgewicht 400 g/mol) | ad 100 g |
| Gesamtmenge Mittel (b) | 100 g |

| Mittel (c) | (c) |
|---|---|
| Silberpigment (Alegrace Marvelous D 12/77-1 Shiny Silver, 10 %ig) | 1,0 g |
| Gesamtmenge Mittel (c) | 1 g |

### 2. Herstellung der Anwendungsmischung durch Vermischen von (a) und (b)

Das Mittel (b) wurde in eine Applikatorflasche mit Spitze gefüllt. Danach wurde das jeweilige Mittel (a) hinzugefügt. Die Applikatorflasche wurd mit einer verschlossenen Spitze versehen und gesschüttelt.

| AWM 1, Vergleich | AWM 2, Erfindung |
|---|---|
| 0,75 g Mittel (aV) | 7,5 g Mittel (aE) |
| 100 g Mittel (b) | 100 g Mittel (b) |
| kein Vermischen möglich, da das Aminosilikon sich in der Applikatorspitze absetzte | homogene Mischung |

### 3. Herstellung der Anwendungsmischung durch Vermischen von (a) und (b) und (c)

Das Mittel (b) wurde in eine Applikatorflasche mit Spitze gefüllt. Danach wurde das jeweilige Mittel (a) hinzugefügt. Anschließend wurde das Mittel (c) hinzugefügt.

Die Applikatorflasche wurd mit einer verschlossenen Spitze versehen und gesschüttelt.

| AWM 3, Vergleich | AWM 4, Erfindung |
|---|---|
| 0,75 g Mittel (aV) | 7,5 g Mittel (aE) |
| 100 g Mittel (b) | 100 g Mittel (b) |
| 1 g Mittel (c) | 1 g Mittel (c) |
| kein Vermischen möglich, da das Aminosilikon sich in der Applikatorspitze absetzte | homogene Mischung |

## Patentansprüche

1. Verfahren zur Behandlung von keratinischem Material, insbesondere menschlichen Haaren, umfassend die folgenden Schritte:
(1) Bereitstellung eines Mittels (a), wobei das Mittel (a) enthält:
(a1) mindestens ein aminofunktionalisiertes Silikonpolymer, und
(a2) mindestens ein bei 20 °C flüssiges kosmetisches Öl, das von (a1) verschieden ist,
(2) Bereitstellung eines Mittels (b), wobei das Mittel (b) enthält:
(b1) Wasser und/oder mindestens Alkylenglycol der Formel (AG) 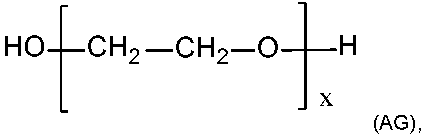 wobei
x für eine ganze Zahl von 1 bis 10000 steht,
(3) gegebenenfalls Bereitstellung eines Mittels (c), wobei das Mittel (c) enthält:
(c1) mindestens ein Pigment,
(4) Herstellung einer Anwendungsmischung durch Vermischen der Mittel (a) und (b) und gegebenenfalls (c),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) gegebenenfalls Ausspülen der Anwendungsmischung.

2. Verfahren nach Anspruch 1, **dadurch gekennzeichnet, dass** das Mittel (a) mindestens ein aminofunktionalisiertes Silikonpolymer (a1) mit mindestens einer sekundären Aminogruppe enthält.

3. Verfahren nach einem der Ansprüche 1 bis 2, **dadurch gekennzeichnet, dass** das Mittel (a) mindestens ein aminofunktionalisiertes Silikonpolymer (a1) enthält, das mindestens eine Struktureinheit der Formel (Si-Amino) umfasst, 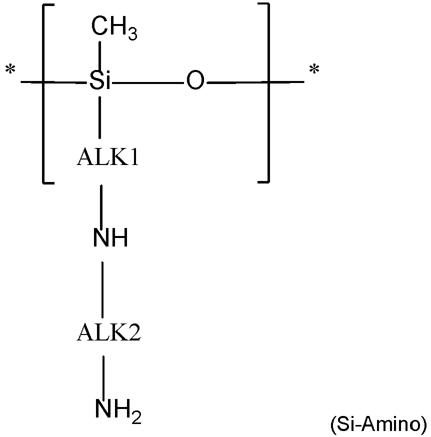 wobei
ALK1 und ALK2 unabhängig voneinander für eine lineare oder verzweigte, zweiwertige C₁-C₂₀-Alkylengruppe stehen.

4. Verfahren nach einem der Ansprüche 1 bis 3, **dadurch gekennzeichnet, dass** das Mittel (a) mindestens ein aminofunktionalisiertes Silikonpolymer (a1) enthält, das Struktureinheiten der Formel (Si-I) und der Formel (Si-II) umfasst

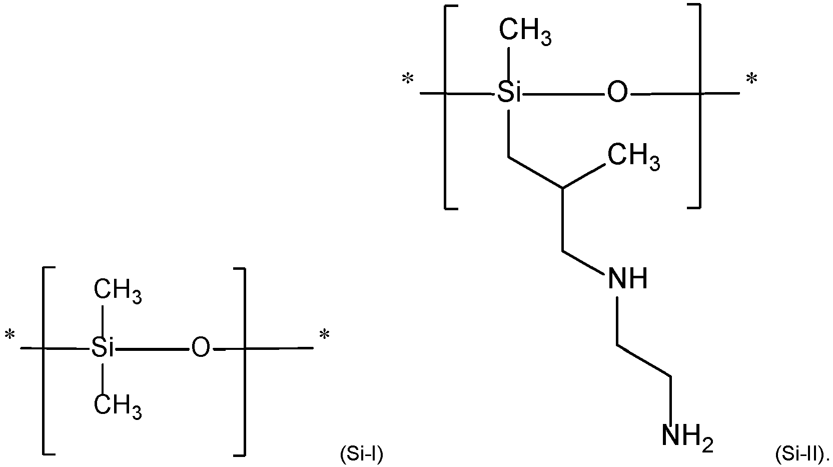

5. Verfahren nach einem der Ansprüche 1 bis 4, **dadurch gekennzeichnet, dass** das Mittel (a) - bezogen auf das Gesamtgewicht des Mittels (a) - ein oder mehrere aminofunktionalisierte Silikonpolymere (a1) in einer Gesamtmenge von 1 bis 90 Gew.-%, bevorzugt von 2 bis 70 Gew.-%, weiter bevorzugt von 3 bis 50 Gew.-%, noch weiter bevorzugt von 4 bis 30 Gew.-%, und ganz besonders bevorzugt von 5 bis 20 Gew.-% enthält.

6. Verfahren nach einem der Ansprüche 1 bis 5, **dadurch gekennzeichnet, dass** das Mittel (a) mindestens ein bei 20 °C flüssiges kosmetisches Öl (a2) enthält, das ausgewählt ist aus der Gruppe aus nicht-aminofunktionalisierten Oligoalkylsiloxanen, nicht-aminofunktionalisierten Silikonpolymeren, Paraffinölen, Isoparaffinölen, synthetischen C₃-C₁₂-Kohlenwasserstoffen, Di-C₁₂-C₂₃-Alkylethern, pflanzlichen Ölen und Esterölen.

7. Verfahren nach einem der Ansprüche 1 bis 6, dadurch gekennzeichet, dass das Mittel (a) mindestens ein bei 20 °C flüssiges kosmetisches Öl (a2) enthält, das ausgewählt ist aus der Gruppe der Oligoalkylsiloxane der Formel (OAS-I) und/oder (OAS-II), 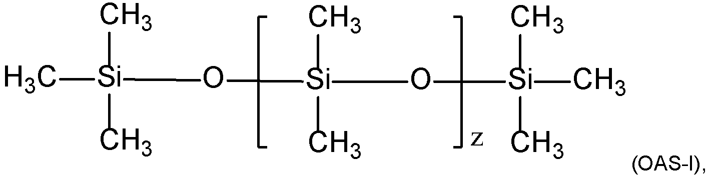 wobei z für eine ganze Zahl von 0 bis 10, bevorzugt für eine ganze Zahl von 0 bis 3, besonders bevorzugt für die Zahl 0 steht, 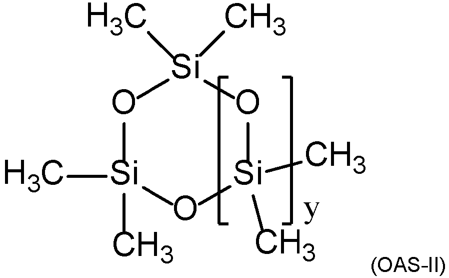 wobei y für eine ganze Zahl von 1 bis 5, bevorzugt für eine ganze Zahl von 1 bis 3, steht.

8. Verfahren nach einem der Ansprüche 1 bis 7, dadurch gekennzeichet, dass das Mittel (a) mindestens ein bei 20 °C flüssiges kosmetisches Öl (a2) enthält, das aus der Gruppe der Polydimethylsiloxane ausgewählt ist und das bevorzugt eine Viskosität von 0,5 bis 30000 mm²/s, weiter bevorzugt von 0,5 bis 20000 mm²/s, noch weiter bevorzugt von 0,5 bis 10000 mm²/s, und besonders bevorzugt von 0,5 bis 500 mm²/s, gemessen nach dem ASTM-Standard D-445, besitzt.

9. Verfahren nach einem der Ansprüche 1 bis 8, **dadurch gekennzeichnet, dass** das Mittel (a) - bezogen auf das Gesamtgewicht des Mittels (a) - ein oder mehrere bei 20 °C flüssige kosmetische Öle in einer Gesamtmenge von 1 bis 99 Gew.-%, bevorzugt von 30 bis 98 Gew.-%, weiter bevorzugt von 50 bis 97 Gew.-%, noch weiter bevorzugt von 70 bis 96 Gew.-% und ganz besonders bevorzugt von 80 bis 95 Gew.-% enthält.

10. Verfahren nach einem der Ansprüche 1 bis 9, **dadurch gekennzeichnet, dass** das Mittel (a) die kosmetischen Öle (a2) und die aminofunktionalisierten Silikonpolymere (a1), d.h. die Bestandteile (a2)/(a1), in einem Gewichtsverhältnis von 100:1 bis 1:100, bevorzugt von 100:1 bis 1:1, weiter bevorzugt von 50:1 bis 2:1, noch weiter bevorzugt von 40:1 bis 4:1 und ganz besonders bevorzugt von 20:1 bis 4:1 enthält.

11. Verfahren nach einem der Ansprüche 1 bis 10, **dadurch gekennzeichnet, dass** das Mittel (b) - bezogen auf das Gesamtgewicht des Mittels (b) - ein oder mehrere Alkylenglycole (b1) der Formel (AG) in einer Gesamtmenge von 10,0 bis 99,0 Gew.-%, bevorzugt von 30,0 bis 95,0 Gew.-%, weiter bevorzugt von 40,0 bis 90,0 Gew.-% und ganz besonders bevorzugt von 40,0 bis 80,0 Gew.-% enthält.

12. Verfahren nach einem der Ansprüche 1 bis 11, **dadurch gekennzeichnet, dass** es ein Verfahren zum Färben von keratinischem Material, insbesondere menschlichen Haaren, ist und dass das Mittel (b) enthält:
(b2) mindestens ein Pigment.

13. Verfahren nach einem der Ansprüche 1 bis 12, **dadurch gekennzeichnet, dass** es ein Verfahren zum Färben von keratinischem Material, insbesondere menschlichen Haaren, ist, und weiterhin **gekennzeichnet durch** die
(3) Bereitstellung eines Mittels (c), wobei das Mittel (c) enthält:
(c1) mindestens ein Pigment, und die
(4) Herstellung einer Anwendungsmischung durch Vermischen der Mittel (a) und (b) und (c).

14. Verfahren nach einem der Ansprüche 1 bis 13, **dadurch gekennzeichnet, dass** das Mittel (b) enthält:
(b3) mindestens einen alkoxylierten Fettsäureester der allgemeinen Formel (AFE-I) 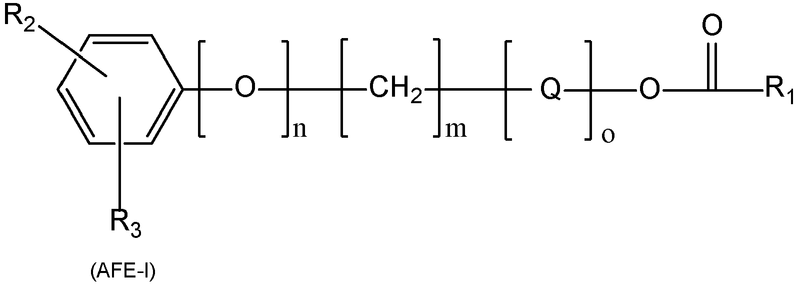 wobei
R1 für eine gesättigte oder ungesättigte C₁₁-C₂₉-Alkylgruppe steht,
R2, R3 unabhängig voneinander für ein Wasserstoffatom, eine C₁-C₆-Alkylgruppe, eine Hydroxygruppe oder eine C₁-C₆-Alkoxygruppe stehen,
n für die Zahl 0 oder 1 steht,
m für eine ganze Zahl von 0 bis 6 steht,
o für eine ganze Zahl von 1 bis 60 steht, und
Q für eine Struktureinheit -O-CH₂-CH₂- , -O-CH(CH₃)-CH₂- oder -O-CH₂-CH(CH₃)- steht,
wobei die Maßgabe besteht, dass, wenn m gleich 0 ist, auch n gleich 0 ist.

15. Verfahren nach einem der Ansprüche 1 bis 14, **dadurch gekennzeichnet, dass** das Mittel (b) enthält:
(b4) mindestens ein Anlagerungsprodukt von C₁-C₆-Alkylenoxid(en) an aliphatische C₁-C₂₄-Alkanole der Formel (AA-I), 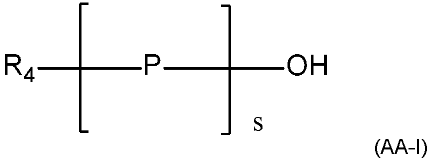 worin
R4 für eine gesättigte oder ungesättigte C₁-C₂₄-Alkylgruppe, bevorzugt für eine C₁-C₁₂-Alkylgruppe, besonders bevorzugt für eine C₁-C₆-Alkylgruppe, steht, und
P für eine Struktureinheit -O-CH₂-CH₂- , -O-CH(CH₃)-CH₂- oder -O-CH₂-CH(CH₃)-steht, und
s für eine ganze Zahl von 1 bis 60, bevorzugt für eine ganze Zahl von 1 bis 40, weiter bevorzugt für eine ganze Zahl von 10 bis 30 und ganz besonders bevorzugt für eine ganze Zahl von 10 bis 20, steht.

16. Verfahren nach einem der Ansprüche 1 bis 15, **gekennzeichnet durch** die
(3) Herstellung einer Anwendungsmischung durch Vermischen der Mittel (a) und (b) und gegebenenfalls (c), wobei die Mittel (a) und (b) in einem Gewichtsverhältnis (a)/(b) von 1:1 bis 1:100, bevorzugt von 1:3 bis 1:50, weiter bevorzugt von 1:5 bis 1:40, noch weiter bevorzugt von 1:7 bis 1:30 und ganz besonders bevorzugt von 1:9 bis 1:28 miteinander vermischt werden.

17. Mehrkomponenten-Verpackungseinheit (Kit-of-parts) zur Behandlung von keratinischem Material, insbesondere menschlichen Haaren, umfassend getrennt voneinander konfektioniert
- einen ersten Container mit einem Mittel (a), wobei das Mittel (a) enthält:
(a1) mindestens ein aminofunktionalisiertes Silikonpolymer, und
(a2) mindestens ein bei 20 °C flüssiges kosmetisches Öl, das von (a1) verschieden ist, und
- einen zweiten Container mit einem Mittel (b), wobei das Mittel (b) enthält:
(b1) Wasser und/oder mindestens Alkylenglycol der Formel (AG) 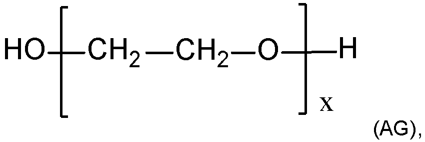 wobei
x für eine ganze Zahl von 1 bis 10000 steht, und
- gegebenenfalls einen dritten Container mit einem Mittel (c), wobei das Mittel (c) enthält:
(c1) mindestens ein Pigment,
wobei die Mittel (a), (b) und (c) und die Bestandteile (a1), (a2), (b1) und (c1) in den Ansprüchen 1 bis 15 definiert sind.

## Claims

1. A method for treating keratinous material, in particular human hair, comprising the following steps:
(1) providing an agent (a), wherein agent (a) contains:
(a1) at least one amino-functionalized silicone polymer, and
(a2) at least one cosmetic oil that is liquid at 20 °C and is different from (a1),
(2) providing an agent (b), wherein agent (b) contains:
(b1) water and/or at least alkylene glycol of the formula (AG) 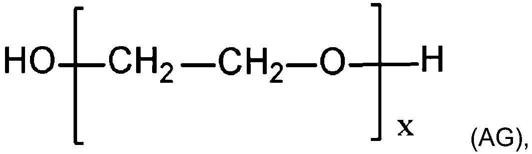 where
x represents an integer from 1 to 10000,
(3) optionally providing an agent (c), wherein agent (c) contains:
(c1) at least one pigment,
(4) preparing an application mixture by mixing agents (a) and (b) and optionally (c),
(5) applying the application mixture prepared in step (4) to the keratinous material,
(6) exposing the keratinous material to the application mixture applied in step (5), and
(7) optionally rinsing out the application mixture.

2. The method according to claim 1, **characterized in that** agent (a) contains at least one amino-functionalized silicone polymer (a1) having at least one secondary amino group.

3. The method according to one of claims 1 to 2, **characterized in that** agent (a) contains at least one amino-functionalized silicone polymer (s1) comprising at least one structural unit of formula (Si-amino), 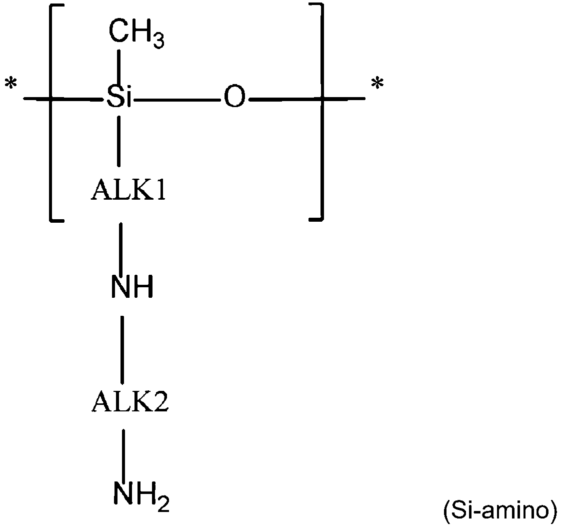 where
ALK1 and ALK2 represent, independently of one another, a linear or branched, divalent C₁-C₂₀ alkylene group.

4. The method according to one of claims 1 to 3, **characterized in that** agent (a) contains at least one amino-functionalized silicone polymer (a1) comprising structural units of formula (Si-I) and of formula (Si-II)

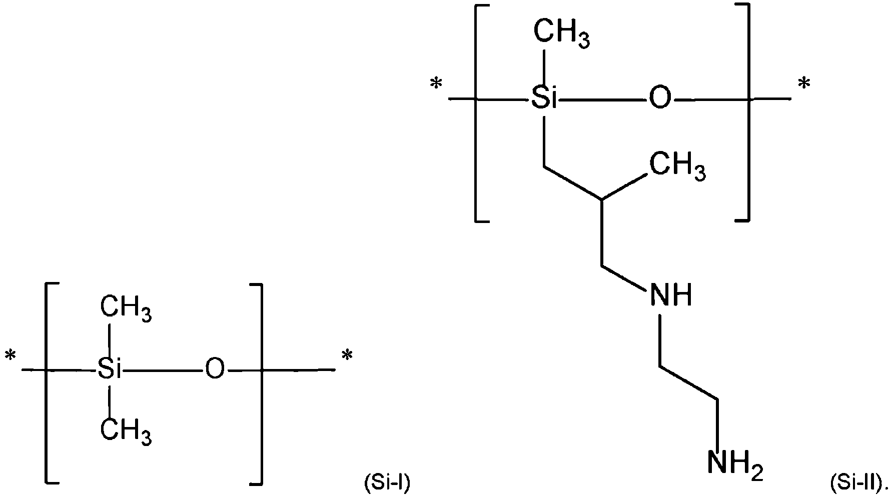

5. The method according to one of claims 1 to 4, **characterized in that** agent (a), based on the total weight of agent (a), contains one or more amino-functionalized silicone polymers (a1) in a total amount from 1 to 90 wt.%, preferably 2 to 70 wt.%, more preferably 3 to 50 wt.%, even more preferably 4 to 30 wt.%, and very particularly preferably 5 to 20 wt.%.

6. The method according to one of claims 1 to 5, **characterized in that** agent (a) contains at least one cosmetic oil (a2) that is liquid at 20 °C and is selected from the group consisting of non-amino-functionalized oligoalkylsiloxanes, non-amino-functionalized silicone polymers, paraffin oils, isoparaffin oils, synthetic C₃-C₁₂ hydrocarbons, Di-C₁₂-C₂₃ alkyl ethers, vegetable oils, and ester oils.

7. The method according to one of claims 1 to 6, **characterized in that** agent (a) contains at least one cosmetic oil (a2) that is liquid at 20 °C and is selected from the group consisting of the oligoalkylsiloxanes of formula (OAS-I) and/or (OAS-II), 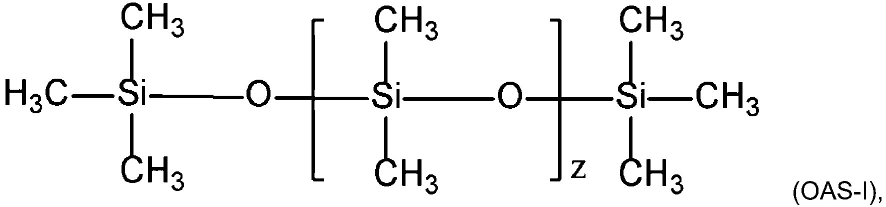 where z represents an integer from 0 to 10, preferably an integer from 0 to 3, particularly preferably 0, 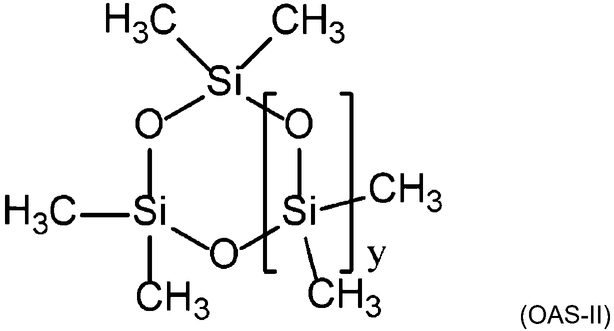 where y represents an integer from 1 to 5, preferably an integer from 1 to 3.

8. The method according to one of claims 1 to 7, **characterized in that** agent (a) contains at least one cosmetic oil (a2) which is liquid at 20 °C and is selected from the group consisting of polydimethylsiloxanes and which preferably has a viscosity of 0.5 to 30000 mm²/s, more preferably 0.5 to 20000 mm²/s, even more preferably 0.5 to 10000 mm²/s, and particularly preferably 0.5 to 500 mm²/s, measured according to ASTM standard D-445.

9. The method according to one of claims 1 to 8, **characterized in that** agent (a) contains, based on the total weight of agent (a), one or more cosmetic oils that are liquid at 20 °C in a total amount of 1 to 99 wt.%, preferably 30 to 98 wt.%, more preferably 50 to 97 wt.%, even more preferably 70 to 96 wt.%, and very particularly preferably 80 to 95 wt.%.

10. The method according to any one of claims 1 to 9,
**characterized by the fact that** agent (a) contains the cosmetic oils (a2) and the amino-functionalized silicone polymers (a1), i.e. the components (a2)/(a1), in a weight ratio of 100:1 to 1:100, preferably 100:1 to 1:1, more preferably 50:1 to 2:1, even more preferably 40:1 to 4:1 and very particularly preferably 20:1 to 4:1.

11. The method according to one of claims 1 to 10, **characterized in that** agent (b) contains, based on the total weight of agent (b), one or more alkylene glycols (b1) of formula (AG) in a total amount of 10.0 to 99.0 wt.%, preferably 30.0 to 95.0 wt.%, more preferably 40.0 to 90.0 wt.%, and very particularly preferably 40.0 to 80.0 wt.%.

12. The method according to one of claims 1 to 11, **characterized in that** it is a method for dyeing keratinous material, in particular human hair, **and in that** agent (b) contains:
(b2) at least one pigment.

13. The method according to one of claims 1 to 12, **characterized in that** it is a method for dyeing keratinous material, in particular human hair, and further **characterized by** the steps of
(3) providing an agent (c), wherein agent (c) contains:
(c1) at least one pigment, and
(4) preparing an application mixture by mixing agents (a) and (b) and (c).

14. The method according to one of claims 1 to 13, **characterized in that** agent (b) contains:
(b3) at least one alkoxylated fatty acid ester of general formula (AFE-I) 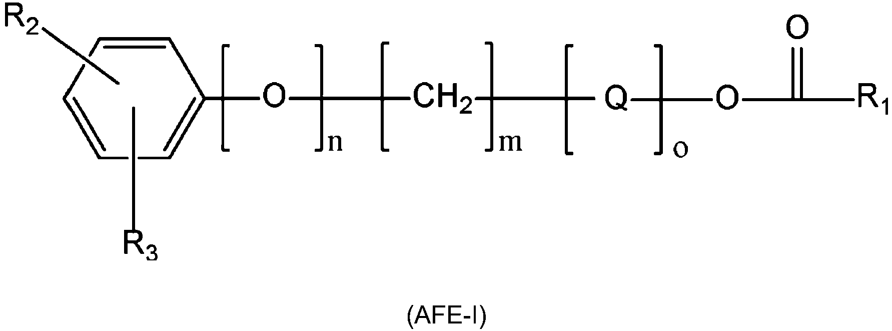 where
R1 represents a saturated or unsaturated C₁₁-C₂₉ alkyl group,
R1, R3 represent, independently of one another, a hydrogen atom, a C₁-C₆ alkyl group, a hydroxy group, or a C₁-C₆ alkoxy group,
n represents the number 0 or 1,
m represents an integer from 0 to 6,
o represents an integer from 1 to 60, and
Q represents a structural unit -O-CH₂-CH₂-, -O-CH(CH₃)-CH₂- or -O-CH₂-CH(CH₃)-,
with the proviso that if m is equal to 0, n is also equal to 0.

15. The method according to one of claims 1 to 14, **characterized in that** agent (b) contains:
(b4) at least one addition product of C₁-C₆ alkylene oxide(s) to aliphatic C₁-C₂₄ alkanols of formula (AA-I), 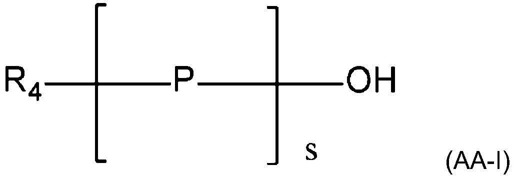 where
R4 represents a saturated or unsaturated C₁-C₂₄ alkyl group, preferably a C₁-C₁₂ alkyl group, particularly preferably a C₁-C₆ alkyl group, and
P represents a structural unit -O-CH₂-CH₂-, -O-CH(CH₃)-CH₂- or -O-CH₂-CH(CH₃)-, and
s represents an integer from 1 to 60, preferably an integer from 1 to 40, more preferably an integer from 10 to 30, and very particularly preferably an integer from 10 to 20.

16. The method according to one of claims 1 to 15, **characterized by** the following step
(3) preparing an application mixture by mixing agents (a) and (b) and optionally (c), agents (a) and (b) being mixed with one another in a weight ratio (a)/(b) of 1:1 to 1:100, preferably 1:3 to 1:50, more preferably 1:5 to 1:40, even more preferably 1:7 to 1:30, and very particularly preferably 1:9 to 1:28.

17. A multi-component packaging unit (kit-of-parts) for treating keratinous material, in particular human hair, comprising separately packaged components - a first container having an agent (a), wherein agent (a) contains:
(a1) at least one amino-functionalized silicone polymer, and
(a2) at least one cosmetic oil that is liquid at 20 °C and is different from (a1), and
- a second container having an agent (b), wherein agent (b) contains:
(b1) water and/or at least alkylene glycol of the formula (AG) 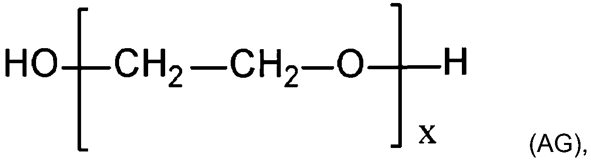 where
x represents an integer from 1 to 10000, and
- optionally a third container having an agent (c), wherein agent (c) contains:
(c1) at least one pigment,
wherein agents (a), (b) and (c) and components (a1), (a2), (b1) and (c1) are defined in claims 1 to 15.

## Revendications

1. Procédé permettant le traitement d'une matière kératinique, en particulier de cheveux humains, comprenant les étapes suivantes :
(1) fourniture d'un agent (a), dans lequel l'agent (a) contient :
(a1) au moins un polymère de silicone aminofonctionnalisé, et
(a2) au moins une huile cosmétique liquide à 20 °C qui est différente de (a1),
(2) fourniture d'un agent (b), dans lequel l'agent (b) contient :
(b1) de l'eau et/ou au moins un alkylène glycol de formule (AG) 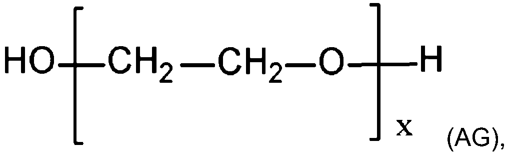 où
x représente un nombre entier allant de 1 à 10 000,
(3) éventuellement, fourniture d'un agent (c), dans lequel l'agent (c) contient :
(c1) au moins un pigment,
(4) préparation d'un mélange à appliquer en mélangeant les agents (a) et (b) et éventuellement (c),
(5) application du mélange à appliquer préparé à l'étape (4) sur la matière kératinique,
(6) attente de l'action du mélange à appliquer appliqué à l'étape (5) sur la matière kératinique, et
(7) éventuellement, élimination par rinçage du mélange à appliquer.

2. Procédé selon la revendication 1, **caractérisé en ce que** l'agent (a) contient au moins un polymère de silicone aminofonctionnalisé (a1) comportant au moins un groupe amine secondaire.

3. Procédé selon l'une des revendications 1 à 2, **caractérisé en ce que** l'agent (a) contient au moins un polymère de silicone aminofonctionnalisé (a1) qui comprend au moins un motif structural de formule (Si-Amino), 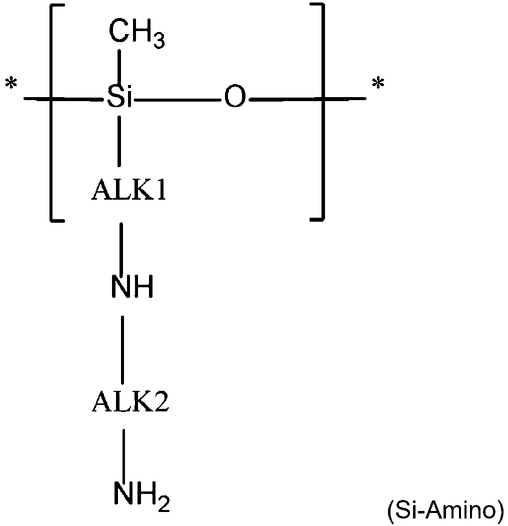 où
ALK1 et ALK2 représentent, indépendamment l'un de l'autre, un groupe alkylène en C₁-C₂₀ bivalent, linéaire ou ramifié.

4. Procédé selon l'une des revendications 1 à 3, **caractérisé en ce que** l'agent (a) contient au moins un polymère de silicone aminofonctionnalisé (a1) qui comprend des motifs structuraux de formule (Si-I) et de formule (Si-II)

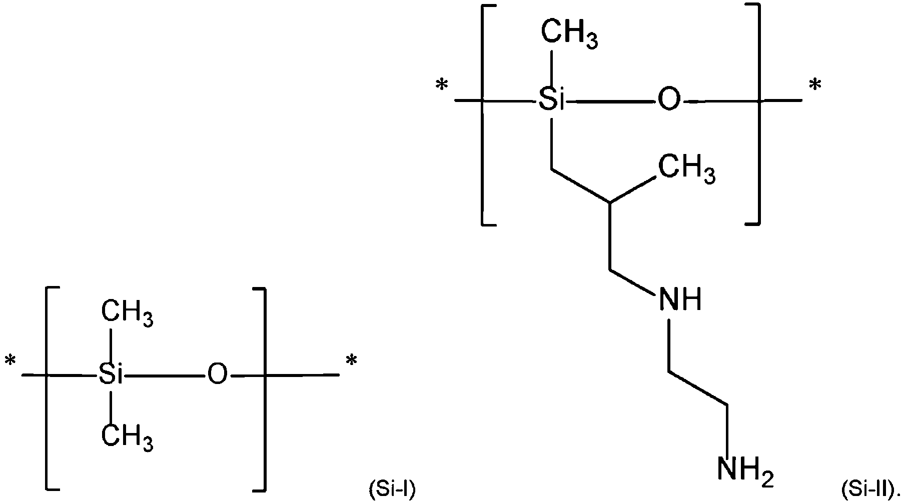

5. Procédé selon l'une des revendications 1 à 4, **caractérisé en ce que** l'agent (a) contient, par rapport au poids total de l'agent (a), un ou plusieurs polymères de silicone aminofonctionnalisés (a1) en une quantité totale allant de 1 à 90 % en poids, de préférence de 2 à 70 % en poids, plus préférablement de 3 à 50 % en poids, encore plus préférablement de 4 à 30 % en poids et de manière tout particulièrement préférée de 5 à 20 % en poids.

6. Procédé selon l'une des revendications 1 à 5, **caractérisé en ce que** l'agent (a) contient au moins une huile cosmétique liquide à 20 °C (a2), choisie dans le groupe constitué d'oligoalkylsiloxanes non aminofonctionnalisés, polymères de silicone non aminofonctionnalisés, huiles de paraffine, huiles d'isoparaffine, hydrocarbures synthétiques en C₃-C₁₂, éthers de dialkyle en C₁₂-C₂₃, huiles végétales et huiles esters.

7. Procédé selon l'une des revendications 1 à 6, **caractérisé en ce que** l'agent (a) contient au moins une huile cosmétique liquide à 20 °C (a2), choisie dans le groupe des oligoalkylsiloxanes de formule (OAS-I) et/ou (OAS-II), 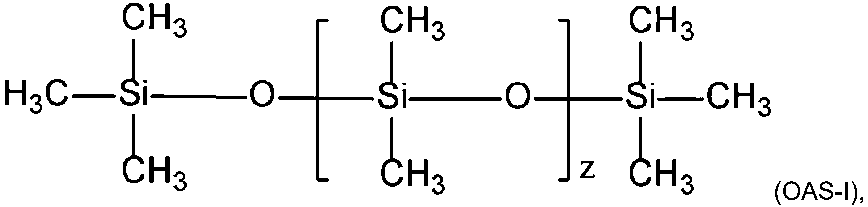 où z représente un nombre entier allant de 0 à 10, de préférence un nombre entier allant de 0 à 3, de manière particulièrement préférée le nombre 0, 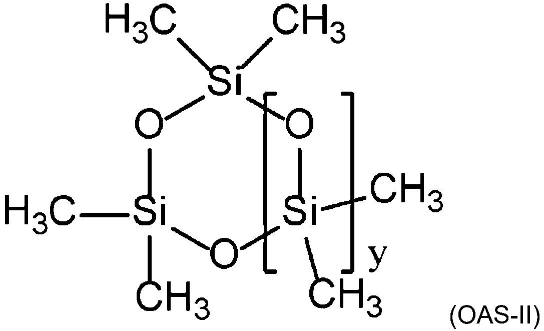 où y représente un nombre entier allant de 1 à 5, de préférence un nombre entier allant de 1 à 3.

8. Procédé selon l'une des revendications 1 à 7, **caractérisé en ce que** l'agent (a) contient au moins une huile cosmétique liquide à 20 °C (a2), choisie dans le groupe des polydiméthylsiloxanes et possédant de préférence une viscosité allant de 0,5 à 30 000 mm²/s, plus préférablement de 0,5 à 20 000 mm²/s, encore plus préférablement de 0,5 à 10 000 mm²/s et de manière particulièrement préférée de 0,5 à 500 mm²/s, mesurée selon la norme ASTM D-445.

9. Procédé selon l'une des revendications 1 à 8, **caractérisé en ce que** l'agent (a) contient, par rapport au poids total de l'agent (a), une ou plusieurs huiles cosmétiques liquides à 20 °C en une quantité totale allant de 1 à 99 % en poids, de préférence de 30 à 98 % en poids, plus préférablement de 50 à 97 % en poids, encore plus préférablement de 70 à 96 % en poids et de manière tout particulièrement préférée de 80 à 95 % en poids.

10. Procédé selon l'une des revendications 1 à 9, **caractérisé en ce que** l'agent (a) contient les huiles cosmétiques (a2) et les polymères de silicone aminofonctionnalisés (a1), c'est-à-dire les constituants (a2)/(a1), dans un rapport pondéral allant de 100:1 à 1:100, de préférence de 100:1 à 1:1, plus préférablement de 50:1 à 2:1, encore plus préférablement de 40:1 à 4:1 et de manière tout particulièrement préférée de 20:1 à 4:1.

11. Procédé selon l'une des revendications 1 à 10, **caractérisé en ce que** l'agent (b) contient, par rapport au poids total de l'agent (b), un ou plusieurs alkylène glycols (b1) de formule (AG) en une quantité totale allant de 10,0 à 99,0 % en poids, de préférence de 30,0 à 95,0 % en poids, plus préférablement de 40,0 à 90,0 % en poids et de manière tout particulièrement préférée de 40,0 à 80,0 % en poids.

12. Procédé selon l'une des revendications 1 à 11, **caractérisé en ce qu'il** s'agit d'un procédé permettant la coloration de matière kératinique, en particulier de cheveux humains, **et en ce que** l'agent (b) contient :
(b2) au moins un pigment.

13. Procédé selon l'une des revendications 1 à 12, **caractérisé en ce qu'il** s'agit d'un procédé permettant la coloration de matière kératinique, en particulier de cheveux humains, et en outre **caractérisé par**
(3) la fourniture d'un agent (c), dans lequel l'agent (c) contient :
(c1) au moins un pigment, et
(4) la préparation d'un mélange à appliquer en mélangeant les agents (a) et (b) et (c).

14. Procédé selon l'une des revendications 1 à 13, **caractérisé en ce que** l'agent (b) contient :
(b3) au moins un ester d'acide gras alcoxylé de formule générale (AFE-I) 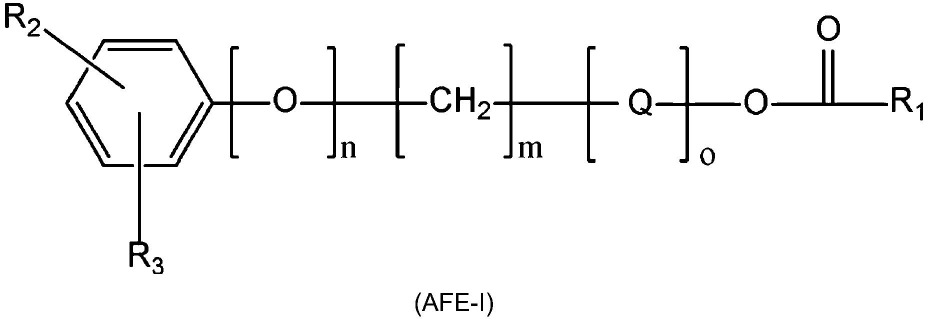 où
R1 représente un groupe alkyle en C₁₁-C₂₉ saturé ou insaturé,
R2, R3 représentent, indépendamment l'un de l'autre, un atome d'hydrogène, un groupe alkyle en C₁-C₆, un groupe hydroxy ou un groupe alcoxy en C₁-C₆,
n représente le nombre 0 ou 1,
m représente un nombre entier allant de 0 à 6,
o représente un nombre entier allant de 1 à 60, et
Q représente un motif structural -O-CH₂-CH₂-, -O-CH(CH₃)-CH₂- ou -O-CH₂-CH(CH₃)-,
dans lequel, à condition que, si m est égal à 0, n soit également égal à 0.

15. Procédé selon l'une des revendications 1 à 14, **caractérisé en ce que** l'agent (b) contient :
(b4) au moins un produit d'addition d'oxyde(s) d'alkylène en C₁-C₆ à des alcanols aliphatiques en C₁-C₂₄ de formule (AA-I), 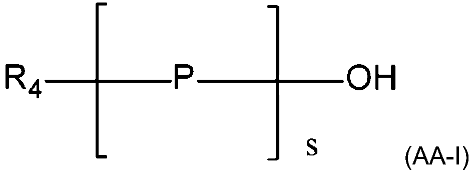 où
R4 représente un groupe alkyle en C₁-C₂₄ saturé ou insaturé, de préférence un groupe alkyle en C₁-C₁₂, de manière particulièrement préférée un groupe alkyle en C₁-C₆, et
P représente un motif structural -O-CH₂-CH₂-, -O-CH(CH₃)-CH₂- ou -O-CH₂-CH(CH₃)-, et
s représente un nombre entier allant de 1 à 60, de préférence un nombre entier allant de 1 à 40, plus préférablement un nombre entier allant de 10 à 30 et de manière tout particulièrement préférée un nombre entier allant de 10 à 20.

16. Procédé selon l'une des revendications 1 à 15, **caractérisé par**
(3) la préparation d'un mélange à appliquer par mélange des agents (a) et (b) et éventuellement (c), dans lequel les agents (a) et (b) sont mélangés entre eux dans un rapport pondéral (a)/(b) allant de 1:1 à 1:100, de préférence de 1:3 à 1:50, plus préférablement de 1:5 à 1:40, encore plus préférablement de 1:7 à 1:30 et de manière tout particulièrement préférée de 1:9 à 1:28.

17. Unité d'emballage à plusieurs composants (kit de pièces) pour le traitement de matière kératinique, en particulier de cheveux humains, comprenant, confectionnés séparément les uns des autres, un premier récipient comportant un agent (a), dans laquelle l'agent (a) contient :
(a1) au moins un polymère de silicone aminofonctionnalisé, et
(a2) au moins une huile cosmétique liquide à 20 °C qui est différente de (a1), et
- un deuxième récipient comportant un agent (b), dans laquelle l'agent (b) contient :
(b1) de l'eau et/ou au moins un alkylène glycol de formule (AG) 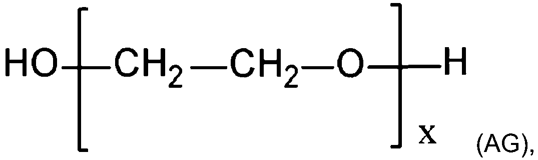 où
x représente un nombre entier allant de 1 à 10 000, et
- éventuellement, un troisième récipient comportant un agent (c), dans laquelle l'agent (c) contient :
(c1) au moins un pigment,
dans laquelle les agents (a), (b) et (c) et les constituants (a1), (a2), (b1) et (c1) sont définis dans les revendications 1 à 15.
